# EUROPEAN PATENT APPLICATION

(11) **EP 4 011 367 A1**
(43) Date of publication of application: **15.06.2022**
(21) Application number: 20212914.4
(22) Date of filing: 09.12.2020
(51) Int. Cl.: A61K 31/05, A61K 31/145, A61K 31/18, A61K 31/275, A61K 31/305, A61K 31/352, A61K 31/353, A61K 31/4035, A61K 31/41, A61K 31/4164, A61K 31/428, A61K 31/433, A61K 31/4439, A61K 31/444, A61K 31/473, A61K 31/496, A61K 31/5377, A61K 31/555, A61P 11/00, A61P 31/14

(54) **COMPOUNDS FOR USE IN THE TREATMENT OF COVID-19**

(71) Applicant: Dompe' Farmaceutici S.P.A., 20122 Milan (IT); Fraunhofer-Gesellschaft zur Förderung der angewandten Forschung e.V., 80686 München (DE)
(72) Inventor: BECCARI, Andrea Rosario, 80131 Napoli (IT); MANELFI, Candida, 80131 Napoli (IT); TALARICO, Carmine, 80131 Napoli (IT); Iaconis, Daniela, 80131 Napoli (IT); ZALIANI, Andrea, 22359 Hamburg (DE); GEISSLINGER, Gerd, 60596 Frankfurt am Main (DE); GRIBBON, Philip, 22587 Hamburg (DE); Claussen, Carsten, 80686 München (DE); Zuzikov, Maria, 80686 München (DE)
(74) Representative: Mauri, Elisabetta Maria Ester

(57) **Abstract**

The present invention relates to compounds for the treatment of a SARS-Cov-2 infection in a subject.

## Description

### FIELD OF THE INVENTION

The present invention relates to compounds that are able to inhibit replication of SARS-CoV-2 virus and thus are useful in the treatment of SARS-CoV-2 virus infections.

### STATE OF THE ART

Coronaviruses (Covs) are a large family of viruses belonging to the family Coronaviridae. The limited number of coronaviruses known to be circulating in humans were considered to cause mild infections and they were regarded in the past as relatively harmless respiratory human pathogens. However, in the last years the emergence of the severe acute respiratory syndrome coronavirus (SARS-CoV) and the Middle East Respiratory Syndrome (MERS) virus revealed that some coronaviruses could cause severe and sometimes fatal respiratory tract infections in humans (Pereira, H et al., 1989 Coronaviridae. Andrewes 'Viruses of Vertebrates, 5th ed.pp. 42-57; Holmes, K.V. et al., 1996. Virology 1, 1075-1093). The first known case of SARS-CoV occurred in Foshan, China in November 2002 and new cases emerged in mainland China in February 2003. The first emergence of MERS-CoV occurred in June 2012 in Saudi Arabia. These events demonstrated that the threats of CoVs should not be underestimated and that it is of paramount importance to advance the knowledge on the replication of these viruses and their interactions with the hosts to develop treatments and vaccines.

In December 2019, atypical pneumonia cases emerged in China and the cause was identified as being a novel coronavirus named SARS-CoV-2 by WHO.

Investigations of the epidemiological and clinical characteristics and outcomes of patients infected by SARS-CoV-2 demonstrated that the infection caused clusters of severe respiratory illness similar to the known SARS-CoV. Furthermore, it was demonstrated that the SARS-CoV-2 can cause severe illness in some patients, even initially it did not transmit readily between people. However, more recent epidemiological data suggest the new virus has undergone human host adaptation/evolution and has become more efficient in human-to-human transmission. Analysis of SARS-CoV-2 genome sequences obtained from patients during the beginning of the outbreak demonstrated that they are almost identical to each other and share 79.5% sequence identity to SARS-CoV. Furthermore, the SARS-CoV-2 is 96% identical at the whole genome level to a bat coronavirus. Phylogenetic analysis of CoVs of different species indicated that SARS-CoV-2 could have originated from Chinese horseshoe bats, but the intermediate transmission vehicle has not yet been identified (Dong, N. et al. Microbiology 2020). According to this study, SARS-CoV-2 belongs to a novel type of bat coronavirus owing to a high degree of variation from the human SARS virus. SARS-CoV-2 is the seventh member of the family of CoVs that infect humans.

Like SARS-CoV, SARS-CoV-2 enters target cells through an endosomal pathway and uses the same cell entry receptor, Angiotensin-converting enzyme II (ACE2).

An effective therapeutic target in SARS-CoV and several other CoVs, are replication-related enzymes, such as proteases (Zhou, P. et al. Microbiology 104, 2020). Drugs that inhibit conserved proteases are capable of preventing replication and proliferation of the virus by interfering with the post-translational processing of essential viral polypeptides. They can also reduce the risk of mutation-mediated drug resistance. This was the case for the SARS-CoV, as inhibitors targeting the main protease involved in replication and proliferation were the most effective means to alleviate the epidemic.

However, the design and development of new drugs is a lengthy process that is not thus adequate to face the emergency of the immediate global challenge of COVID-19 outbreak.

An alternative, approach that is more efficient is the repurposing of drugs already tested as safe in man or approved for different therapeutic applications. This is a rapid response solution, since the pharmacokinetic, toxicological, and manufacturing data for the drugs are already available, thus allowing immediate application in clinical setting. 2006;6(4):361-76) (Anand et al., Science 2003;300(5626):1763-7).

Therefore, computational drug repurposing is an effective approach to rapidly identify and select drugs safe in man that are promising candidates in the treatment of SARS-Cov-2

### SUMMARY OF THE INVENTION

The applicants have carried out the analysis of a library containing more than 10,000 commercialized drugs and clinical candidates "safe in man" or characterized up to late clinical stage and have selected by Computer-Aided Drug Design a number of molecules able to bind to SARS-Cov-2 functional proteins essential for the pathogenetic mechanisms. Furthermore, the applicants have confirmed by in vitro screening the ability of the selected molecules to inhibit virus survival and replication in infected host cells.

In details, the compounds selected are better described below, wherein also the chemical name and formula for each compound are provided.
Compounds of formula (I) 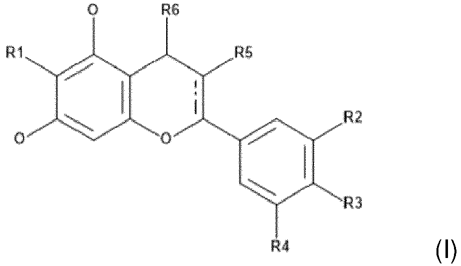 wherein
R1, R2, R3 and R4 are independently selected from OH and H,
R5 is selected from the group consisting of OH, H alpha-L-rhamnopyranosidyl and 3,4,5-trihydroxybenzoate
R6 is selected from H and carbonyl,
with the proviso that at least one of the following condition is satisfied:
R1 is OH, or
R2, R3, R4 are OH or
R3 is OH and the fused rings are a chromone;
compounds of formula (II) 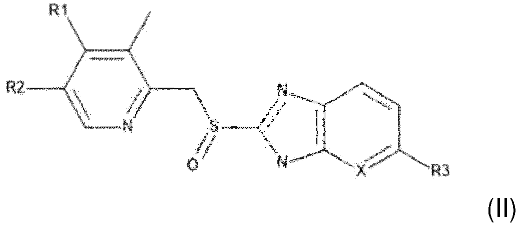 wherein
R1 is selected from the group consisting of OCH₂CF₃, OCH₂CH₂CH₂OCH₃ and OCH₃,
R2 is selected from H and CH₃,
R3 is selected from H and OCH₃,
X is selected from N and C;
compounds of formula (III) 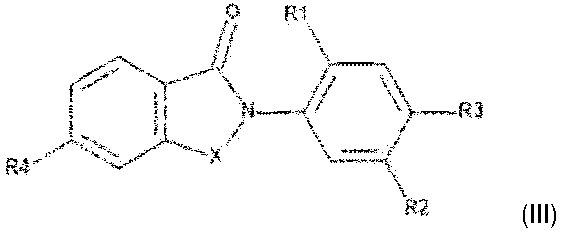 wherein
R1 is selected from H and CH₃,
R2 is selected from the group consisting of H, CH₃ and SO₂N(CH₃)₂,
R3 is selected from H and CH₃,
R4 is selected from H and F,
X is selected from S and Se;
compounds of formula (IV) 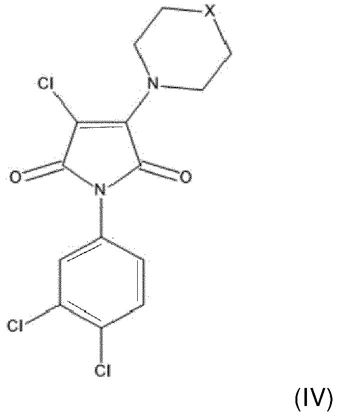 wherein
X is selected from O and NCH₃;
compounds of formula (V) 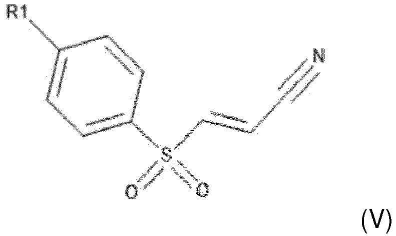 wherein
R1 is selected from CH₃ and C(CH₃)₃;
compounds of formula (VI) 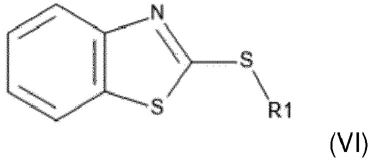 wherein
R1 is selected from the group consisting of H, N-Cyclohexyl, S-benzothiazol-2-yl and morpholin-4-yl;
compounds of formula (VII) 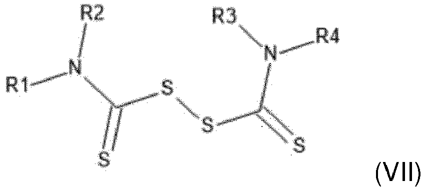 wherein R1, R2, R3 and R4 are independently selected from CH₃ and CH₂CH₃; compounds of formula (VIII) 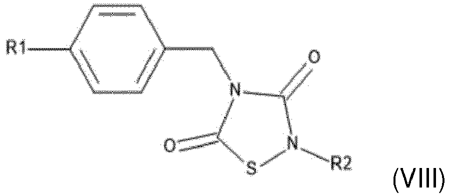 wherein
R1 is selected from H and F,
R2 is selected from 4-methylphenyl and 1-naphthalenyl;
compounds of formula (IX) 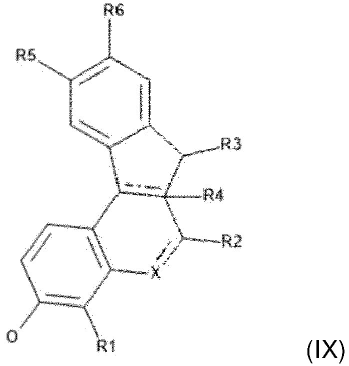 wherein
R1, R4, R5, R6 are independently selected from H and OH,
R2 is selected from H and NCH₂CH₂N(CH₃)₂,
R3 is selected from H and carbonyl,
X is selected from N and O;
the compounds listed in the table below:

| **N.** | **Chemical structure** | **Current Name** | **Chemical Name** |
|---|---|---|---|
| 19 | 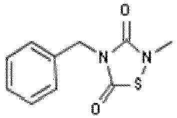 | TZDZ-8 | 4-Benzyl-2-methyl-1,2,4-thiadiazolidine-3,5-dione |
| 20 | 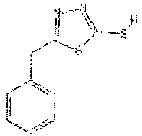 | CL-17107 | 5-benzyl-3H-1,3,4-thiadiazole-2-thione |
| 21 | 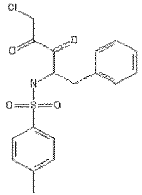 | Tosyl Phenanyl Chloromethyl Ketone | N-[(2S)-5-chloro-3,4-dioxo-1-phenylpentan-2-yl]-4-methylbenzenesulfonamide |
| 22 | 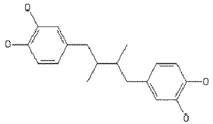 | Nordihydroguaia retic acid | 4-[4-(3,4-dihydroxyphenyl)-2,3-dimethylbutyl]benzene-1,2-diol |
| 23 | 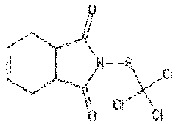 | Captan | 2-(trichloromethylsulfanyl)-3a,4,7,7a-tetrahydroisoindole-1,3-dione |
| 28 | 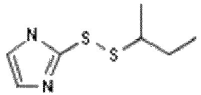 | PX-12 | (2-Imidazolyl)(1-methylpropyl)disulfide |
| 29 | 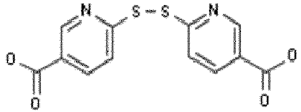 | Carboxypyridinedisulfide | 6-[(5-carboxypyridin-2-yl)disulfanyl]pyridine-3-carboxylic acid |
| 30 | 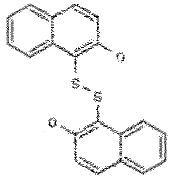 | IPA-3 | 1,1'-Disulfanediylbis(2-naphthol) |
| 31 | 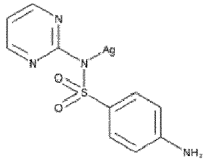 | Silver-sulfadiazine | silver;(4-aminophenyl)sulfonyl-pyrimidin-2-ylazanide |
| 32 | 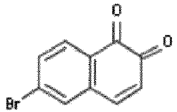 | Bonaphthone | 6-Bromonaphthalene-1,2-dione |
| 33 | 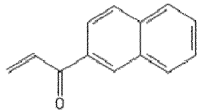 | JAK3-inhibitor-V | 1-naphthalen-2-ylprop-2-en-1-one |
| 34 | 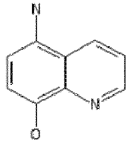 | PD119507 | 5-aminoquinolin-8-ol |
| 35 | 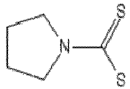 | Pyrrolidine-dithiocarbamate | N-pyrrolidin-1-ylcarbamodithioate |
| 36 | 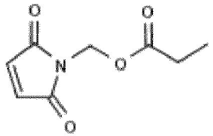 | MIRA-1 | Propionic acid 2,5-dioxo-2,5-dihydro-1H-pyrrol-1-ylmethyl ester |
| 39 | 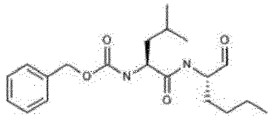 | Calpeptin | N2-[(Benzyloxy)carbonyl]-N-[(2S)-1-oxohexan-2-yl]-L-leucinamide |
| 40 | 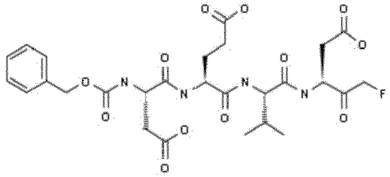 | Z-DEVD-FMK | N-(Benzyloxycarbonyl)-L-aspartyl-L-glutamyl-L-valyl-L-aspartylfluoromethane |
| 41 | 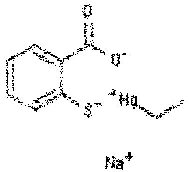 | Thimerosal | 2-Sulfidobenzoic acid ethylmercury(1+) sodium salt |
| 42 | 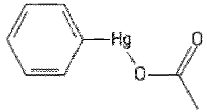 | Phenylmercuric acetate | acetyloxy(phenyl)mercury |
| 43 | 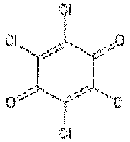 | Chloranil | 2,3,5,6-tetrachlorocyclohexa-2,5-diene-1,4-dione |
| 44 | 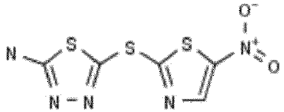 | SU3327 | 5-(5-Nitrothiazol-2-ylsulfanyl)-1,3,4-thiadiazol-2-am ine |
| 47 | 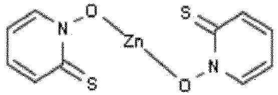 | Pyrithione Zinc | Bis[1-hydroxy-2(1H)-pyiridinethionato]zinc |
| 48 | 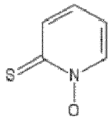 | Pyrithione | 1-hydroxypyridine-2-thione |
| 50 | 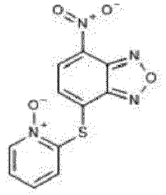 | NSC228155 | 4-Nitro-7-[(1-oxidopyridin-2-yl)sulfanyl]-2,1,3-benzoxadiazole |
| 51 | 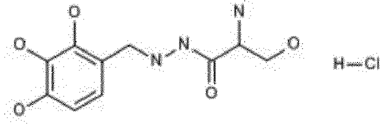 | Benserazide Hydrochloride | 2-Amino-3-hydroxy-N'-[(2,3,4-trihydroxyphenyl)methyl]propan ehydrazide hydrogen chloride |
| 52 | 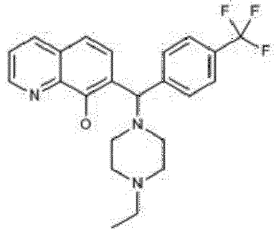 | ML311 | 7-[(4-Ethylpiperazin-1-yl)[4-(trifluoromethyl)phenyl]methyl]q uinolin-8-ol |
| 54 | 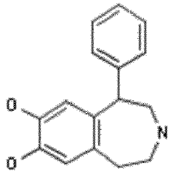 | SKF-38393 | 2,3,4,5,-Tetrahydro-7,8-dihydroxy-1-phenyl-1H-3-benzazepine |
| 55 | 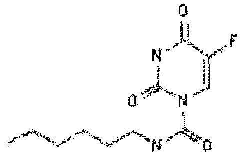 | Carmofur | 5-Fluoro-N-hexyl-2,4-dioxo-1,2,3,4-tetrahydropyrimidine-1-carboxamide |
| 56 | 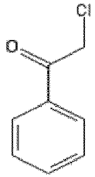 | Phenacyl chloride | 2-chloro-1-phenylethanone |
| 57 | 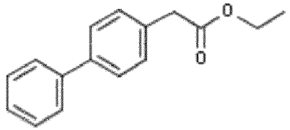 | Felbinac ethyl | [1,1'-Biphenyl]-4-acetic acid ethyl ester |
| 58 | 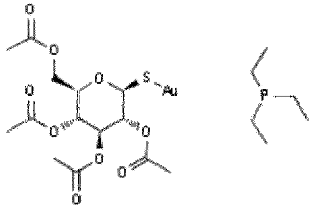 | Auranofin | (2346-Tetra-O-acetyl-1-thio-beta-D-gl ucopyra nosato-S)(triethylphosphine)gold |
| 59 | 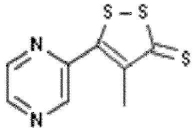 | Oltipraz | 4-Methyl-5-(2-pyrazinyl)-3H-1,2-dithiole-3-thione |
| 60 | 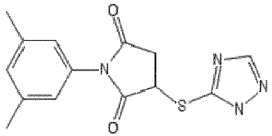 | WAY-308264 | 1-(3,5-dimethylphenyl)-3-(1H-1,2,4-triazol-5-ylsulfanyl)pyrrolidine-2,5-dione |
| 61 | 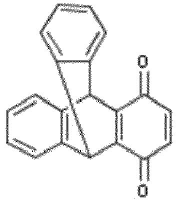 | INCA-6 | Pentacyclo[6.6.6.0(2,7).0(9,14).0( 15,20)]icosa-2(7),4,9,11,13,15,17,19-octaene-3,6-dione |
| 62 | 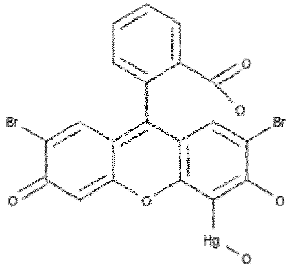 | Spectrum_00051 0 | [2,7-dibromo-9-(2-carboxyphenyl)-3-hydroxy-6-oxoxanthen-4-yl]mercury;hydrate |
| 63 | 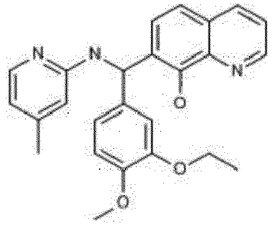 | HQ-415 | 7-[(3-Ethoxy-4-methoxyphenyl)[(4-methylpyridin-2-yl)amino]methyl]quinolin-8-ol |
| 64 | 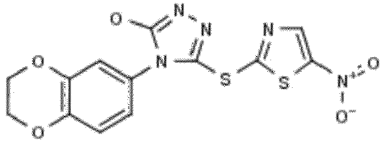 | BI-78D3 | 4-(2,3-Dihydro-1,4-benzodioxin-6-yl)-5-(5-nitrothiazol-2-ylsulfanyl)-4H-1,2,4-triazol-3-ol |
| 65 | 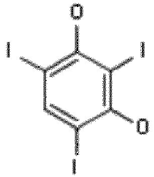 | Riodoxol | 2,4,6-Triiodoresorcine |
| 66 | 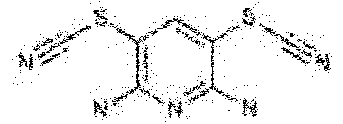 | PR-619 | 2,6-Diaminopyridine-3,5-diyl bis(thiocyanate) |
| 67 | 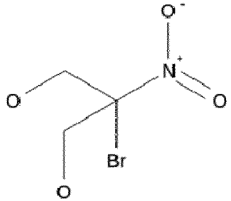 | Bronopol | 2-bromo-2-nitropropane-1,3-diol |
| 68 | 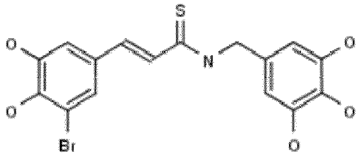 | NT157 | 3-(3-Bromo-4,5-dihydroxyphenyl)-N-(3,4,5-trihydroxybenzyl)-2-propenethioamide |
| 69 | 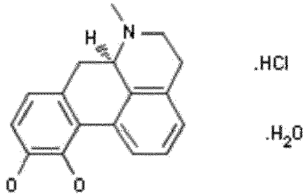 | Apomorphine hydrochloride | (R)-6-Methyl-5,6,6a,7-tetrahydro-4H-dibenzo[de,g]quinoline-10,11-diol hydrochloride hemihydrate |
| 70 | 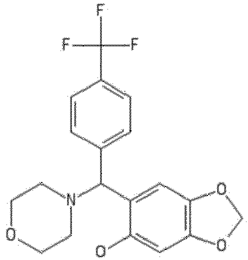 | PD120911 | 6-[morpholin-4-yl-[4-(trifluoromethyl)phenyl]methyl]-1,3-benzodioxol-5-ol |
| 71 | 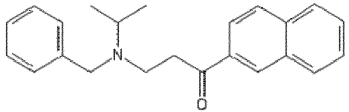 | ZM-39923 | 3-[benzyl(propan-2-yl)amino]-1-naphthalen-2-ylpropan-1-one |
| 72 | 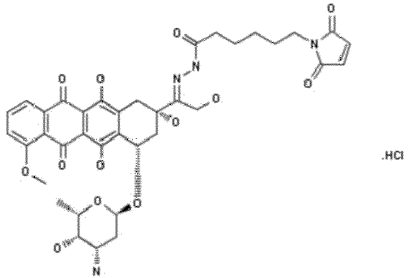 | Aldoxorubicin | N'-[1-[4(S)-(3-Amino-2,3,6-trideoxy-alpha-L-lyxohexopyranosyloxy)-2(S),5,12-trihydroxy-7-methoxy-6,11-dioxo-1,2,3,4,6,11-hexahydronaphthacen-2-yl]-2-hydroxyethylidene]-6-(2,5-dioxo-2,5-dihydro-1H-pyrrol-1-yl)hexanohydrazide hydrochloride |
| 73 | 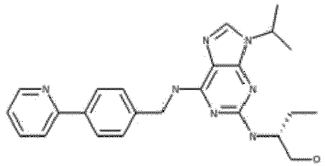 | CR8-(R) | (2R)-2-[[9-propan-2-yl-6-[(4-pyridin-2-ylphenyl)methylamino]purin-2-yl]amino]butan-1-ol |
| 74 | 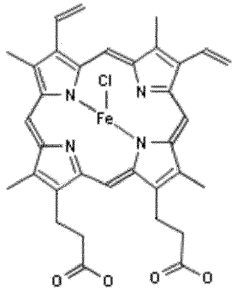 | Hemin | - (SP-5-13)-Chloro[3,8,13,17-tetramethyl-7,12-divinyl-21H,23H-porphine-2,18-dipropanoato(4-)-kappa N21,kappaN22,kappaN23,k appa N24]dihydrogenoferrate(2-) |
| 75 | 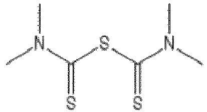 | Tetramethylthiur am monosulfide | dimethylcarbamothioyl N,N-dimethylcarbamodithioate |
| 76 | 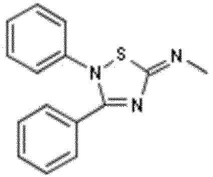 | SCH-202676 | N-(2,3-Diphenyl-2,5-dihydro-1,2,4-thiadiazol-5-ylidene)-N-methylamine |
| 77 | 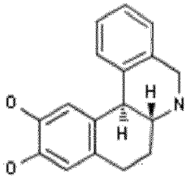 | Dihydrexidine | trans-10,11-Dihydroxy-5,6,6a,7,8,12b-hexahydrobenzo[a]phenanthridi ne |
| 78 | 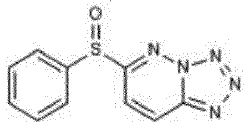 | Ro-106-9920 | 6-(Phenylsulfinyl)tetrazolo[1,5-b]pyridazine |
| 79 | 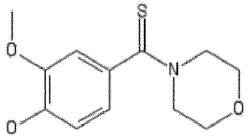 | Vanitiolide | (4-hydroxy-3-methoxyphenyl)-morpholin-4-ylmethanethione |
| 80 | 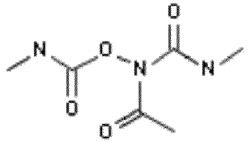 | Caracemide | N-[(Methylamino)carbonyl]-N-[[(methylamino)carbonyl]oxy]ace tamide |
| 81 | 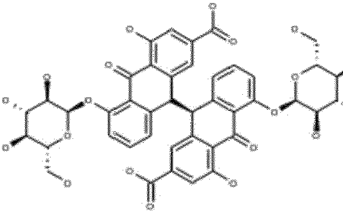 | Sennoside A | (9R)-9-[(9R)-2-carboxy-4-hydroxy-10-oxo-5-[(2S,3R,4S,5S,6R)-3,4,5-trihydroxy-6-(hydroxymethyl)oxan-2-yl]oxy-9H-anthracen-9-yl]-4-hydroxy-10-oxo-5-[(2S,3R,4S,5S,6R)-3,4,5-trihydroxy-6-(hydroxymethyl)oxan-2-yl]oxy-9H-anthracene-2-carboxylic acid |
| 82 | 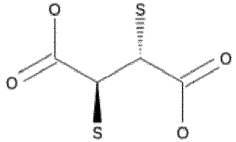 | Dimercaptosucci nic-acid | 2,3-bis(sulfanyl)butanedioic acid |
| 83 | 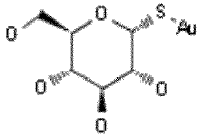 | Aurothioglucose | 1-Aurothioglucose |
| 84 | 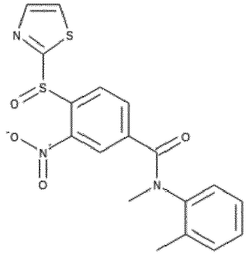 | SB-268262 | N-methyl-N-(2-methylphenyl)-3-nitro-4-(1,3-thiazol-2-ylsulfinyl)benzamide |
| 85 | 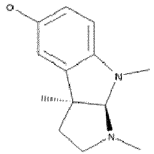 | eseroline-(-) | (3aS,8bR)-3,4,8b-trimethyl-2,3a-dihydro-1H-pyrrolo[2,3-b]indol-7-ol |
| 86 | 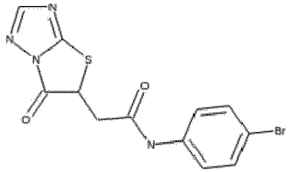 | PD096194 | N-(4-bromophenyl)-2-(6-oxo-[1,3]thiazolo[3,2-b][1,2,4]triazol-5-yl)acetamide |
| 87 | 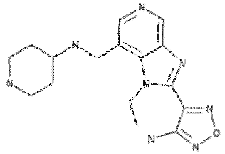 | SB-747651A | 4-[1-ethyl-1-[(piperidin-4-ylamino)methyl]imidazo[4,5-c]pyridin-2-yl]-1,2,5-oxadiazol-3-amine |
| 88 | 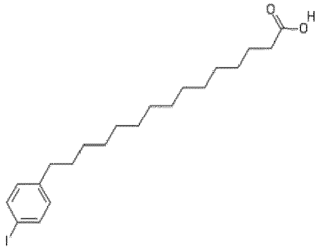 | omega-(4-lodophenyl)pent adecanoic acid | 15-(4-iodophenyl) pentadecanoic acid |
| 89 | 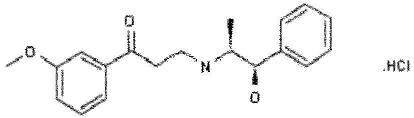 | oxyfedrine | 3-[2(R)-Hydroxy-1(S)-methyl-2-phenylethylamino]-1-(3-methoxyphenyl)propan-1-one hydrochloride |
| 90 | 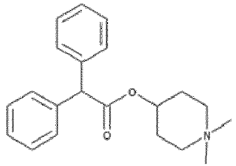 | 4-DAMP | (1,1-dimethylpiperidin-1-ium-4-yl) 2,2-diphenylacetate;iodide |
| 91 | 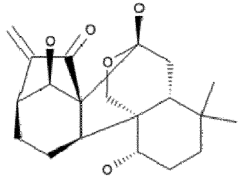 | Oridonin (Isodonol) | (1S,5S,8R,9S,10S,11R,15S,18R)-9,10,15,18-tetrahydroxy-12,12-dimethyl-6-methylidene-17-oxapentacyclo[7.6.2.15,8.01,11.0 2,8]octadecan-7-one |

All these compounds are well known approved drugs or clinical candidates for different therapeutic indications with "safe in man" trials completed.

The inventors have also tested pharmaceutically acceptable salts and hydrate forms of the above compounds, and have confirmed that these also maintain the activity against the virus.

The experimental results obtained by the inventors and described in the Experimental Section below demonstrate that all these compounds are useful for the treatment of a SARS-Cov-2 infection in a subject.

Accordingly, a first object of the invention is a compound selected from the compounds above and pharmaceutically acceptable salt therof, for use in the treatment of a SARS-Cov-2 infection in a subject.

A second object of the invention is a pharmaceutical composition comprising i) a compound selected from the compounds above and pharmaceutically acceptable salts thereof, and ii) at least one inert pharmaceutically acceptable excipient, for use in the treatment of a SARS-Cov-2 infection in a subject.

A third object of the invention is a method of treating of a SARS-Cov-2 infection in a subject, comprising administering the subject a compound selected from the compounds above and pharmaceutically acceptable salts therof.

### DETAILED DESCRIPTION OF THE INVENTION

A first object of the present invention is a compound, for use in the treatment of a SARS-Cov-2 infection in a subject, wherein said compound is selected from:
a compound of formula (I) 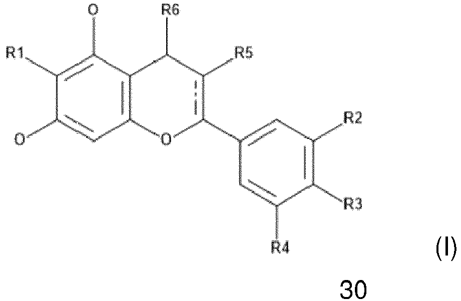 wherein
R1, R2, R3 and R4 are independently selected from OH and H,
R5 is selected from the group consisting of OH, H, alpha-L-rhamnopyranosidyl and 3,4,5-trihydroxybenzoate
R6 is selected from H and carbonyl,
with the proviso that at least one of the following condition is satisfied:
R1 is OH, or
R2, R3, R4 are OH or
R3 is OH and the fused rings are a chromone;
a compound of formula (II) 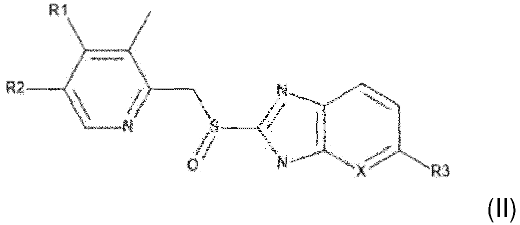 wherein
R1 is selected from the group consisting of OCH₂CF₃, OCH₂CH₂CH₂OCH₃ and OCH₃,
R2 is selected from H and CH₃,
R3 is selected from H and OCH₃,
X is selected from N and C;
a compound of formula (III) 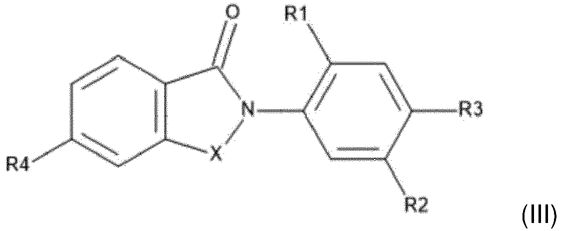 wherein
R1 is selected from H and CH₃,
R2 is selected from the group consisting of H, CH₃ and SO₂N(CH₃)₂,
R3 is selected from H and CH₃,
R4 is selected from H and F,
X is selected from S and Se;
a compound of formula (IV) 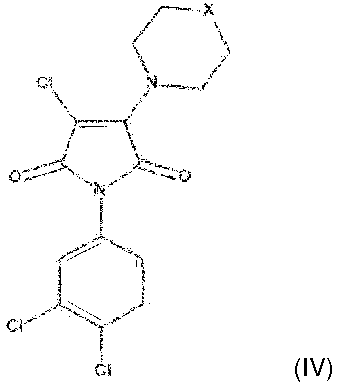 wherein
X is selected from O and NCH₃;
a compound of formula (V) 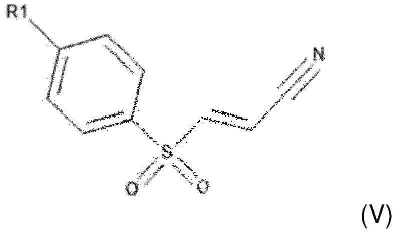 wherein
R1 is selected from CH₃ and C(CH₃)₃;
a compound of formula (VI) 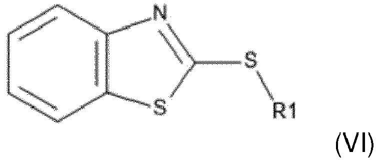 wherein R1 is selected from the group consisting of H, N-Cyclohexyl, S-benzothiazol-2-yl and
morpholin-4-yl;
a compound of formula (VII) 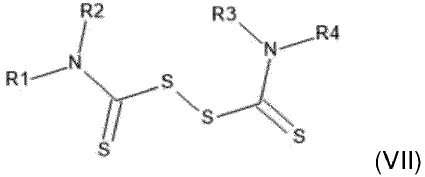 wherein R1, R2, R3 and R4 are independently selected from CH₃ and CH₂CH₃;
a compound of formula (VIII) 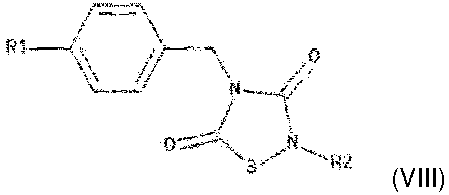 wherein
R1 is selected from H and F,
R2 is selected from 4-methylphenyl and 1-naphthalenyl;
a compound of formula (IX) 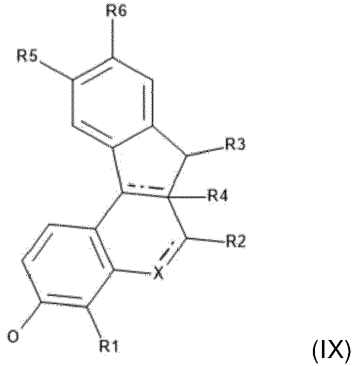 wherein
R1, R4, R5, R6 are independently selected from H and OH,
R2 is selected from H and NCH₂CH₂N(CH₃)₂,
R3 is selected from H and carbonyl,
X is selected from N and O;
and the compounds listed in the table below:

| **N.** | **Chemical structure** | **Current Name** | **Chemical Name** |
|---|---|---|---|
| 19 | 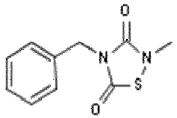 | TZDZ-8 | 4-Benzyl-2-methyl-1,2,4-thiadiazolidine-3,5-dione |
| 20 | 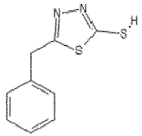 | CL-17107 | 5-benzyl-3H-1,3,4-thiadiazole-2-thione |
| 21 | 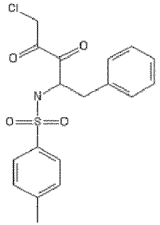 | Tosyl Phenanyl Chloromethyl Ketone | N-[(2S)-5-chloro-3,4-dioxo-1-phenylpentan-2-yl]-4-methylbenzenesulfonamide |
| 22 | 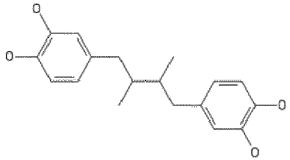 | Nordihydroguaia retic acid | 4-[4-(3,4-dihydroxyphenyl)-2,3-dimethylbutyl]benzene-1,2-diol |
| 23 | 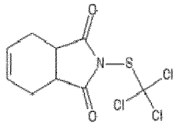 | Captan | 2-(trichloromethylsulfanyl)-3a,4,7,7a-tetrahydroisoindole-1,3-dione |
| 28 | 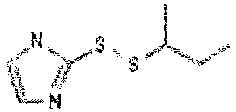 | PX-12 | (2-Imidazolyl)(1-methylpropyl)disulfide |
| 29 | 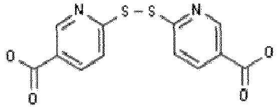 | Carboxypyridinedisulfide | 6-[(5-carboxypyridin-2-yl)disulfanyl]pyridine-3-carboxylic acid |
| 30 | 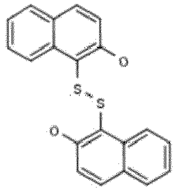 | IPA-3 | 1,1'-Disulfanediylbis(2-naphthol) |
| 31 | 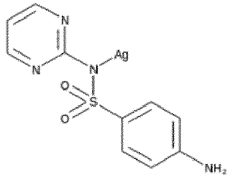 | Silver-sulfadiazine | silver;(4-aminophenyl)sulfonyl-pyrimidin-2-ylazanide |
| 32 | 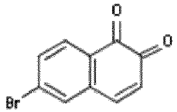 | Bonaphthone | 6-Bromonaphthalene-1,2-dione |
| 33 | 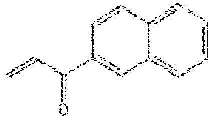 | JAK3-inhibitor-V | 1-naphthalen-2-ylprop-2-en-1-one |
| 34 | 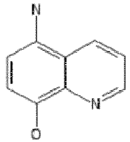 | PD119507 | 5-aminoquinolin-8-ol |
| 35 | 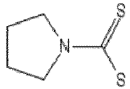 | Pyrrolidine-dithiocarbamate | N-pyrrolidin-1-ylcarbamodithioate |
| 36 | 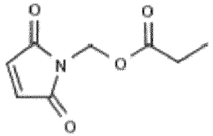 | MIRA-1 | Propionic acid 2,5-dioxo-2,5-dihydro-1H-pyrrol-1-ylmethyl ester |
| 39 | 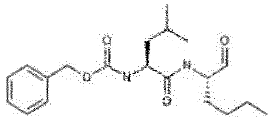 | Calpeptin | N2-[(Benzyloxy)carbonyl]-N-[(2S)-1-oxohexan-2-yl]-L-leucinamide |
| 40 | 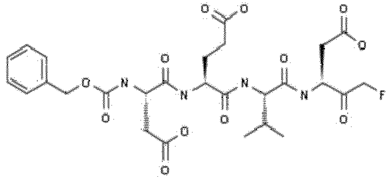 | Z-DEVD-FM K | N-(Benzyloxycarbonyl)-L-aspartyl-L-glutamyl-L-valyl-L-aspartylfluoromethane |
| 41 | 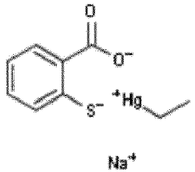 | Thimerosal | 2-Sulfidobenzoic acid ethylmercury(1+) sodium salt |
| 42 | 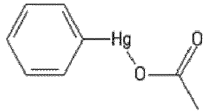 | Phenylmercuric acetate | acetyloxy(phenyl)mercury |
| 43 | 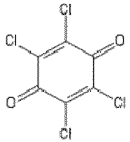 | Chloranil | 2,3,5,6-tetrachlorocyclohexa-2,5-diene-1,4-dione |
| 44 | 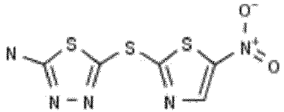 | SU3327 | 5-(5-Nitrothiazol-2-ylsulfanyl)-1,3,4-thiadiazol-2-amine |
| 47 | 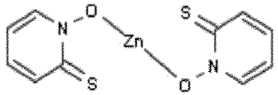 | Pyrithione Zinc | Bis[1-hydroxy-2(1H)-pyiridinethionato]zinc |
| 48 | 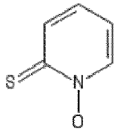 | Pyrithione | 1-hydroxypyridine-2-thione |
| 50 | 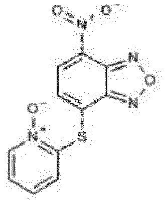 | NSC228155 | 4-Nitro-7-[(1-oxidopyridin-2-yl)sulfanyl]-2,1,3-benzoxadiazole |
| 51 | 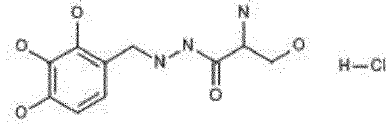 | Benserazide Hydrochloride | 2-Amino-3-hydroxy-N'-[(2,3,4-trihydroxyphenyl)methyl]propan ehydrazide hydrogen chloride |
| 52 | 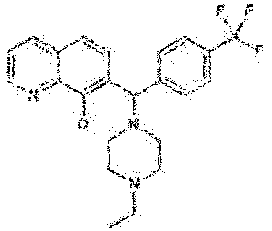 | ML311 | 7-[(4-Ethylpiperazin-1-yl)[4-(trifluoromethyl)phenyl]methyl]q uinolin-8-ol |
| 54 | 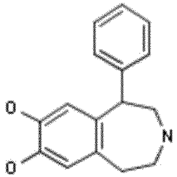 | SKF-38393 | 2,3,4,5,-Tetrahydro-7,8-dihydroxy-1-phenyl-1H-3-benzazepine |
| 55 | 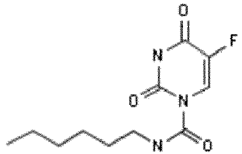 | Carmofur | 5-Fluoro-N-hexyl-2,4-dioxo-1,2,3,4-tetrahydropyrimidine-1-carboxamide |
| 56 | 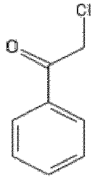 | Phenacyl chloride | 2-chloro-1-phenylethanone |
| 57 | 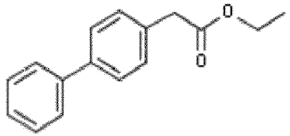 | Felbinac ethyl | [1,1'-Biphenyl]-4-acetic acid ethyl ester |
| 58 | 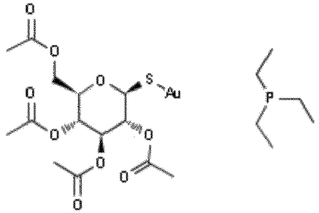 | Auranofin | (2,3,4,6-Tetra-O-acetyl-1-thio*-beta-D-gl ucopyra nosato-S)(triethylphosphine)gold |
| 59 | 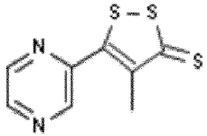 | Oltipraz | 4-Methyl-5-(2-pyrazinyl)-3H-1,2-dithiole-3-thione |
| 60 | 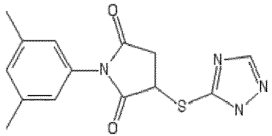 | WAY-308264 | 1-(3,5-dimethylphenyl)-3-(1H-1,2,4-triazol-5-ylsulfanyl)pyrrolidine-2,5-dione |
| 61 | 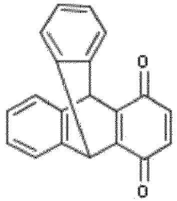 | INCA-6 | Pentacyclo[6.6.6.0(2,7).0(9,14).0( 15,20)]icosa-2(7),4,9,11,13,15,17,19-octaene-3,6-dione |
| 62 | 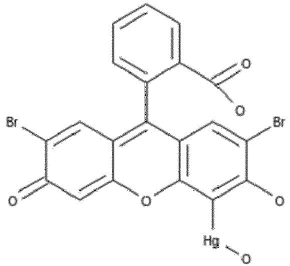 | Spectrum_00051 0 | [2,7-dibromo-9-(2-carboxyphenyl)-3-hydroxy-6-oxoxanthen-4-yl]mercury;hydrate |
| 63 | 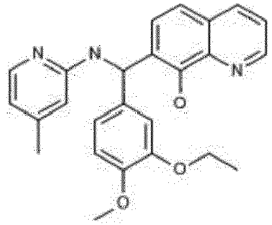 | HQ-415 | 7-[(3-Ethoxy-4-methoxyphenyl)[(4-methylpyridin-2-yl)amino]methyl]quinolin-8-ol |
| 64 | 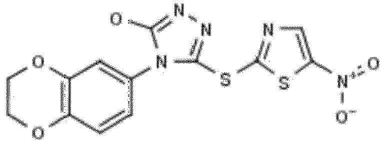 | BI-78D3 | 4-(2,3-Dihydro-1,4-benzodioxin-6-yl)-5-(5-nitrothiazol-2-ylsulfanyl)-4H-1,2,4-triazol-3-ol |
| 65 | 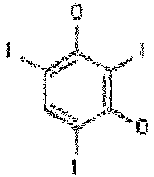 | Riodoxol | 2,4,6-Triiodoresorcine |
| 66 | 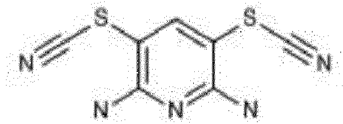 | PR-619 | 2,6-Diaminopyridine-3,5-diyl bis(thiocyanate) |
| 67 | 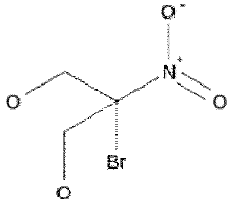 | Bronopol | 2-bromo-2-nitropropane-1,3-diol |
| 68 | 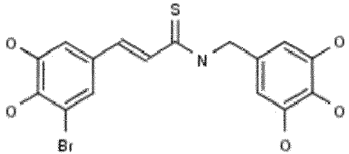 | NT157 | 3-(3-Bromo-4,5-dihydroxyphenyl)-N-(3,4,5-trihydroxybenzyl)-2-propenethioamide |
| 69 | 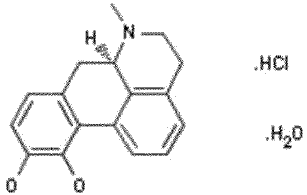 | Apomorphine hydrochloride | (R)-6-Methyl-5,6,6a,7-tetrahydro-4H-dibenzo[de,g]quinoline-10,11-diol hydrochloride hemihydrate |
| 70 | 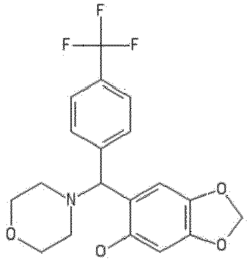 | PD120911 | 6-[morpholin-4-yl-[4-(trifluoromethyl)phenyl]methyl]-1,3-benzodioxol-5-ol |
| 71 | 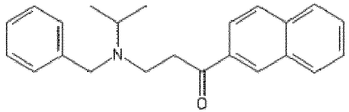 | ZM-39923 | 3-[benzyl(propan-2-yl)amino]-1-naphthalen-2-ylpropan-1-one |
| 72 | 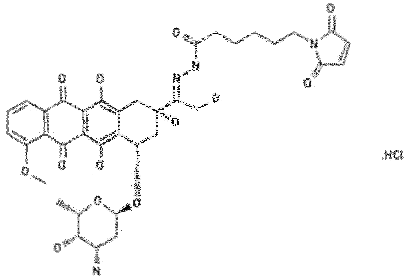 | Aldoxorubicin | N'-[1-[4(S)-(3-Amino-2,3,6-trideoxy-alpha-L-lyxo-hexopyranosyloxy)-2(S),5,12-trihydroxy-7-methoxy-6,11-dioxo-1,2,3,4,6,11-hexahydronaphthacen-2-yl]-2-hydroxyethylidene]-6-(2,5-dioxo-2,5-dihydro-1H-pyrrol-1-yl)hexanohydrazide hydrochloride |
| 73 | 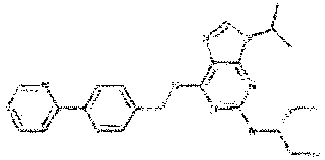 | CR8-(R) | (2R)-2-[[9-propan-2-yl-6-[(4-pyridin-2-ylphenyl)methylamino]purin-2-yl]amino]butan-1-ol |
| 74 | 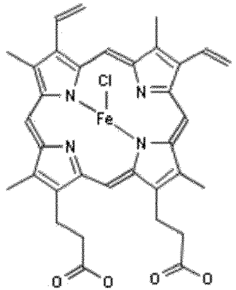 | Hemin | -(SP-5-13)-Chloro[3,8,13,17-tetramethyl-7,12-divinyl-21H,23H-porphine-2,18-dipropanoato(4-)-kappa N21,kappaN22,kappaN23,k appaN24]dihydrogenoferrate(2-) |
| 75 | 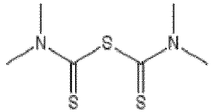 | Tetramethylthiur am monosulfide | dimethylcarbamothioyl N,N-dimethylcarbamodithioate |
| 76 | 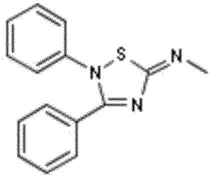 | SCH-202676 | N-(2,3-Diphenyl-2,5-dihydro-1,2,4-thiadiazol-5-ylidene)-N-methylamine |
| 77 | 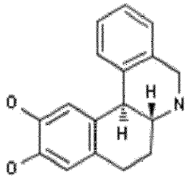 | Dihydrexidine | trans-10,11-Dihydroxy-5,6,6a,7,8,12b-hexahydrobenzo[a]phenanthridi ne |
| 78 | 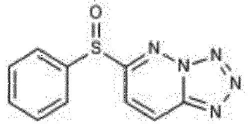 | Ro-106-9920 | 6-(Phenylsulfinyl)tetrazolo[1,5-b]pyridazi ne |
| 79 | 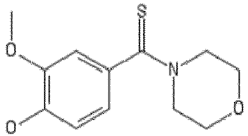 | Vanitiolide | (4-hydroxy-3-methoxyphenyl)-morpholin-4-ylmethanethione |
| 80 | 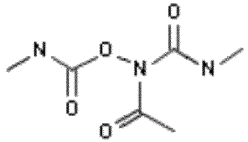 | Caracemide | N-[(Methylamino)carbonyl]-N-[[(methylamino)carbonyl]oxy]ace tamide |
| 81 | 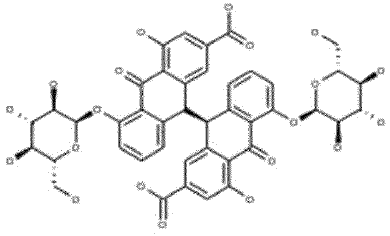 | Sennoside A | (9R)-9-[(9R)-2-carboxy-4-hydroxy-10-oxo-5-[(2S,3R,4S,5S,6R)-3,4,5-trihydroxy-6-(hydroxymethyl)oxan-2-yl]oxy-9H-anthracen-9-yl]-4-hydroxy-10-oxo-5-[(2S,3R,4S,5S,6R)-3,4,5-trihydroxy-6-(hydroxymethyl)oxan-2-yl]oxy-9H-anthracene-2-carboxylic acid |
| 82 | 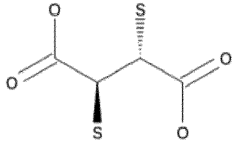 | Dimercaptosucci nic-acid | 2,3-bis(sulfanyl)butanedioic acid |
| 83 | 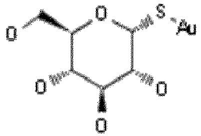 | Aurothioglucose | 1-Aurothioglucose |
| 84 | 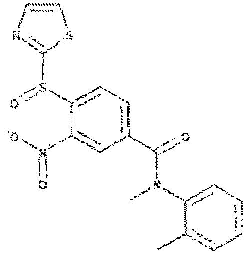 | SB-268262 | N-methyl-N-(2-methylphenyl)-3-nitro-4-(1,3-thiazol-2-ylsulfinyl)benzamide |
| 85 | 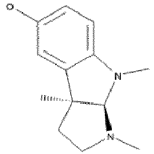 | eseroline-(-) | (3aS,8bR)-3,4,8b-trimethyl-2,3a-dihydro-1H-pyrrolo[2,3-b] indol-7-ol |
| 86 | 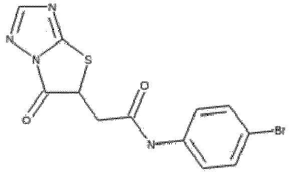 | PD096194 | N-(4-bromophenyl)-2-(6-oxo-[1,3]thiazolo[3,2-b][1,2,4]triazol-5-yl)acetamide |
| 87 | 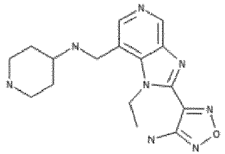 | SB-747651A | 4-[1-ethyl-1-[(piperidin-4-ylamino)methyl]imidazo[4,5-c]pyridin-2-yl]-1,2,5-oxadiazol-3-amine |
| 88 | 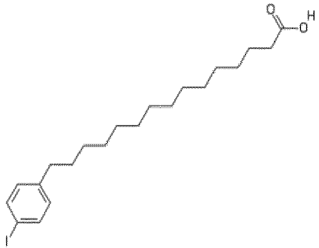 | omega-(4-lodophenyl)pent adecanoic acid | 15-(4-iodophenyl)pentadecanoic acid |
| 89 | 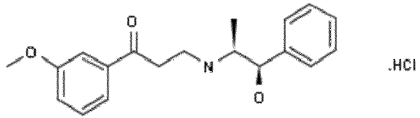 | oxyfedrine | 3-[2(R)-Hydroxy-1(S)-methyl-2-phenylethylamino]-1-(3-methoxyphenyl)propan-1-one hydrochloride |
| 90 | 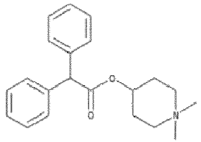 | 4-DAMP | (1,1-dimethylpiperidin-1-ium-4-yl) 2,2-diphenylacetate;iodide |
| 91 | 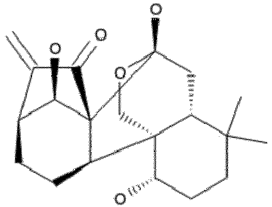 | Oridonin (Isodonol) | (1S,5S,8R,9S,10S,11R,15S,18R)-9,10,15,18-tetrahydroxy-12,12-dimethyl-6-methylidene-17-oxapentacyclo[7.6.2.15,8.01,11.0 2,8]octadecan-7-one |

and pharmaceutically acceptable salts thereof.

As used in the formulas reported herein, a bond represented with a continuous line and a broken line parallel to each other represents a bond that can be a single bond or a double bond.

In a preferred embodiment, said compound of formula (I) is selected from the group consisting of 5,6,7-Trihydroxy-2-phenyl-4H-1-benzopyran-4-one, (+)-3(R),5,7-Trihydroxy-2(R)-(3,4,5-trihydroxyphenyl)-3,4-dihydro-2H-1-benzopyran-4-one, 3,5,7-Trihydroxy-2-(3,4,5-trihydroxyphenyl)-4H-1-benzopyran-4-one, 4',5,7-Trihydroxyflavone, 5,7-Dihydroxy-4-oxo-2-(3,4,5-trihydroxyphenyl)-4H-1-benzopyran-3-yl alpha-L-rhamnopyranoside, (-)-3,4,5-Trihydroxybenzoic acid 5,7-dihydroxy-2(R)-(3,4,5-trihydroxyphenyl)-3,4-dihydro-2H-1-benzopyran-3(R)-yl ester and pharmaceutically acceptable salts thereof.

In a preferred embodiment, said compound of formula (II) is selected from the group consisting of 2-([[3-Methyl-4-(2,2,2-trifluoroethoxy)pyridin-2-yl]methyl]sulfinyl)-1H-benzimidazole, 2-[4-(3-Methoxypropoxy)-3-methylpyridin-2-ylmethylsulfinyl]benzimidazol-1-ide sodium salt, (±)-5-Methoxy-2-(4-methoxy-3,5-dimethylpyridin-2-ylmethylsulfinyl)imidazo[4,5-b]pyridine and pharmaceutically acceptable salts thereof.

In a preferred embodiment, said compound of formula (III) is selected from the group consisting of 2-Phenyl-1,2-benzisoselenazol-3(2H)-one, 2-(2,5-Dimethylphenyl)-6-fluoro-1,2-benzothia zol-3(2H)-one, 2-(4-methylphenyl)-1,2-benzothiazol-3-one, N,N-dimethyl-3-(3-oxo-1,2-benzothiazol-2-yl)benzenesulfonamide, 2-phenyl-1,2-benzothiazol-3-one and pharmaceutically acceptable salts thereof.

In a preferred embodiment, said compound of formula (IV) is selected from 3-chloro-1-(3,4-dichlorophenyl)-4-morpholin-4-ylpyrrole-2,5-dione, 3-chloro-1-(3,4-dichlorophenyl)-4-(4-methylpiperazin-1-yl)pyrrole-2,5-dione and pharmaceutically acceptable salts thereof.

In a preferred embodiment, said compound of formula (V) is selected from 3-(4-Methylphenylsulfonyl)-2-propenenitrile, 3-(4-tert-Butylphenylsulfonyl)-2(E)-propenenitrile and pharmaceutically acceptable salts thereof.

In a preferred embodiment, said compound of formula (VI) is selected from the group consisting of 3H-1,3-benzothiazole-2-thione, N-(1,3-benzothiazol-2-ylsulfanyl)cyclohexanamine, 4-(1,3-benzothiazol-2-ylsulfanyl)morpholine, 2-(1,3-benzothiazol-2-yldisulfanyl)-1,3-benzothiazole and pharmaceutically acceptable salts thereof.

In a preferred embodiment, said compound of formula (VII) is selected from dimethylcarbamothioylsulfanyl N,N-dimethylcarbamodithioate,
Tetraethylthioperoxydicarbonic diamide and pharmaceutically acceptable salts thereof. In a preferred embodiment, said compound of formula (VIII) is selected from 4-Benzyl-2-(1-naphthyl)-1,2,4-thiadiazolidine-3,5-dione, 4-(4-Fluorobenzyl)-2-(4-methylphenyl)-1,2,4-thiadiazolidine-3,5-dione and pharmaceutically acceptable salts thereof.

In a preferred embodiment, said compound of formula (IX) is selected from (6aS,11bR)-7,11b-dihydro-6H-indeno[2,1-c]chromene-3,4,6a,9,10-pentol, 6-[2-(Dimethylamino)ethylamino]-3-hydroxy-7H-indeno[2,1-c]quinolin-7-one dihydrochloride and pharmaceutically acceptable salts thereof.

In a preferred embodiment, the compound for use according to the present invention is selected from the compounds listed in the table below.

| **N.** | **Chemical structure** | **Current Name** | **Chemical Name** |
|---|---|---|---|
| 1 | 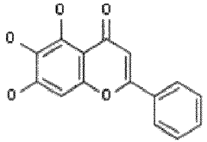 | Baicalein | 5,6,7-Trihydroxy-2-phenyl-4H-1-benzopyran-4-one |
| 2 | 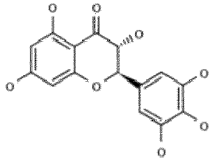 | Dihydromyricetin | (+)-3(R),5,7-Trihydroxy-2(R)-(3,4,5-trihydroxyphenyl)-3,4-dihydro-2H-1-benzopyran-4-one |
| 3 | 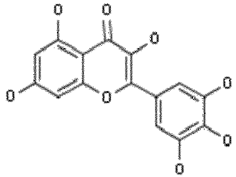 | Myricetin | 3,5,7-Trihydroxy-2-(3,4,5-trihydroxyphenyl)-4H-1-benzopyran-4-one |
| 4 | 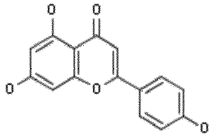 | Apigenin | 4',5,7-Trihydroxyflavone |
| 5 | 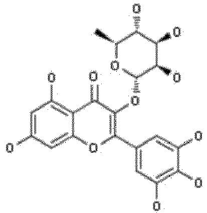 | Myricitrin | 5,7-Dihydroxy-4-oxo-2-(3,4,5-trihydroxyphenyl)-4H-1-benzopyran-3-yl alpha-L-rhamnopyranoside |
| 6 | 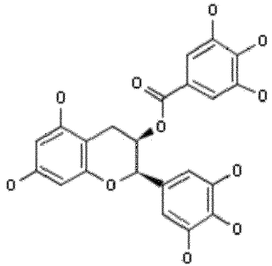 | (-)-Epigallocatechin Gallate | (-)-3,4,5-Trihydroxybenzoic acid 5,7-dihydroxy-2(R)-(3,4,5-trihydroxyphenyl)-3,4-dihydro-2H-1-benzopyran-3(R)-ylester |
| 7 | 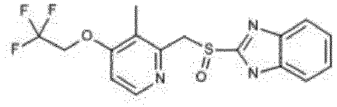 | Lansoprazole | 2-([[3-Methyl-4-(2,2,2-trifluoroethoxy)pyridin-2-yl]methyl]sulfinyl)-1H-benzimidazole |
| 8 | 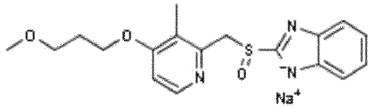 | Rabeprazole (sodium) | 2-[4-(3-Methoxypropoxy)-3-methylpyridin-2-ylmethylsulfinyl]benzimidazol-1-ide sodium salt |
| 9 | 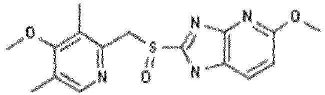 | Tenatoprazole | (±)-5-Methoxy-2-(4-methoxy-3,5-dimethylpyridin-2-ylmethylsulfinyl)imidazo[4,5-b]pyridine |
| 10 | 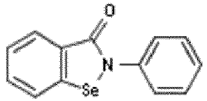 | Ebselen | 2-Phenyl-1,2-benzisoselenazol-3(2H)-one |
| 11 | 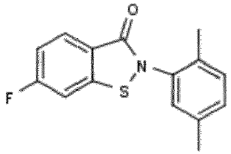 | MLS-0315771 | 2-(2,5-Dimethylphenyl)-6-fluoro-1,2-benzothia zol-3(2H)-one |
| 12 | 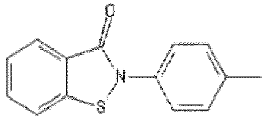 | PBIT | 2-(4-methylphenyl)-1,2-benzothiazol-3-one |
| 13 | 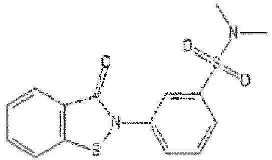 | ML345 Analog | N,N-di methyl-3-(3-oxo-1,2-benzothiazol-2-yl)benzenesulfonamide |
| 14 | 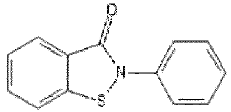 | PD086290 | 2-phenyl-1,2-benzothiazol-3-one |
| 15 | 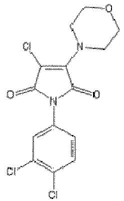 | RI-1 | 3-chloro-1-(3,4-dichlorophenyl)-4-morpholin-4-ylpyrrole-2,5-dione |
| 16 | 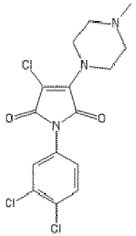 | PD119792 | 3-chloro-1-(3,4-dichlorophenyl)-4-(4-methylpiperazin-1-yl)pyrrole-2,5-dione |
| 17 | 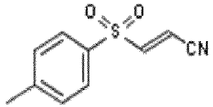 | Bay-11-7082 | 3-(4-Methylphenylsulfonyl)-2-propenenitrile |
| 18 | 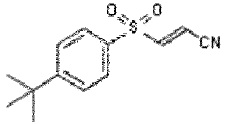 | Bay-11-7085 | 3-(4-tert-Butylphenylsulfonyl)-2(E)-propenenitrile |
| 19 | 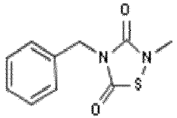 | TZDZ-8 | 4-Benzyl-2-methyl-1,2,4-thiadiazolidine-3,5-dione |
| 20 | 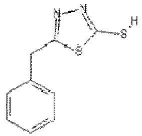 | CL-17107 | 5-benzyl-3H-1,3,4-thiadiazole-2-thione |
| 21 | 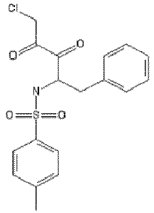 | Tosyl Phenanyl Chloromethyl Ketone | N-[(2S)-5-chloro-3,4-dioxo-1-phenylpentan-2-yl]-4-methylbenzenesulfonamide |
| 22 | 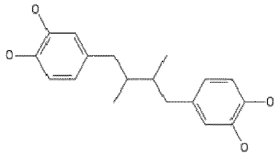 | Nordihydroguaia retic acid | 4-[4-(3,4-dihydroxyphenyl)-2,3-dimethylbutyl]benzene-1,2-diol |
| 23 | 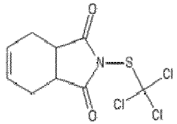 | Captan | 2-(trichloromethylsulfanyl)-3a,4,7,7a-tetrahydroisoindole-1,3-dione |
| 24 | 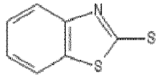 | Benzo[d]thiazole -2(3H)-thione | 3H-1,3-benzothiazole-2-thione |
| 25 | 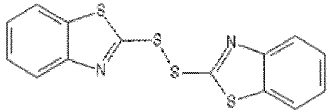 | Benzothiazyl disulfide | 2-(1,3-benzothiazol-2-yldisulfanyl)-1,3-benzothiazole |
| 26 | 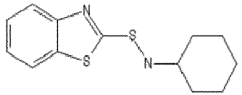 | CBS, N-Cyclohexyl-2-benzothiazolesul fenamide | N-(1,3-benzothiazol-2-ylsulfanyl)cyclohexanamine |
| 27 | 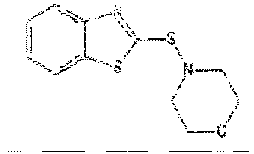 | N-oxydiethylenebe nzothiazole-2-sulfenamide | 4-(1,3-benzothiazol-2-ylsulfanyl)morpholine |
| 28 | 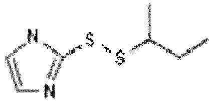 | PX-12 | (2-Imidazolyl)(1-methylpropyl)disulfide |
| 29 | 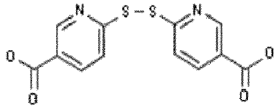 | Carboxypyridinedisulfide | 6-[(5-carboxypyridin-2-yl)disulfanyl]pyridine-3-carboxylic acid |
| 30 | 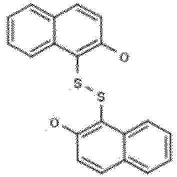 | IPA-3 | 1,1'-Disulfanediylbis(2-naphthol) |
| 31 | 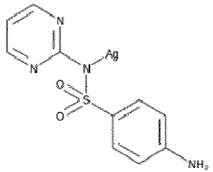 | Silver-sulfadiazine | silver;(4-aminophenyl)sulfonyl-pyrimidin-2-ylazanide |
| 32 | 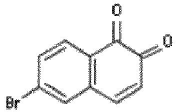 | Bonaphthone | 6-Bromonaphthalene-1,2-dione |
| 33 | 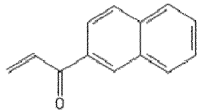 | JAK3-inhibitor-V | 1-naphthalen-2-ylprop-2-en-1-one |
| 34 | 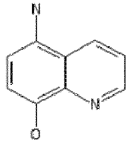 | PD119507 | 5-aminoquinolin-8-ol |
| 35 | 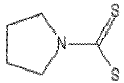 | Pyrrolidine-dithiocarbamate | N-pyrrolidin-1-ylcarbamodithioate |
| 36 | 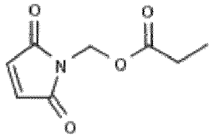 | MIRA-1 | Propionic acid 2,5-dioxo-2,5-dihydro-1H-pyrrol-1-ylmethyl ester |
| 37 | 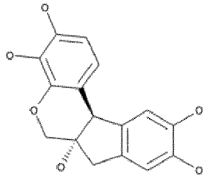 | Hematoxylin (Hydroxybrazilin) | (6aS,11bR)-7,11b-dihydro-6H-indeno[2,1-c]chromene-3,4,6a,9,10-pentol |
| 38 | 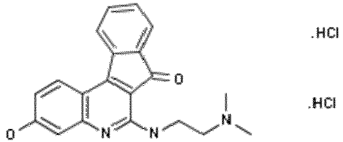 | TAS-103 (dihydrochloride) | 6-[2-(Dimethylamino)ethylamino]-3-hydroxy-7H-indeno[2,1-c]quinolin-7-one dihydrochloride |
| 39 | 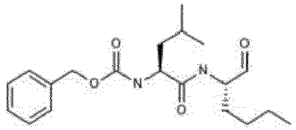 | Calpeptin | N2-[(Benzyloxy)carbonyl]-N-[(2S)-1-oxohexan-2-yl]-L-leucinamide |
| 40 | 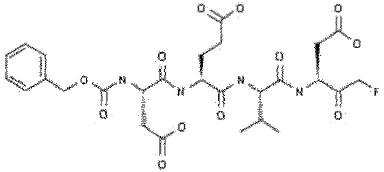 | Z-DEVD-FMK | N-(Benzyloxycarbonyl)-L-aspartyl-L-glutamyl-L-valyl-L-aspartylfluoromethane |
| 41 | 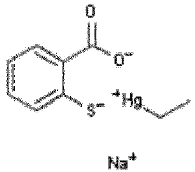 | Thimerosal | 2-Sulfidobenzoic acid ethylmercury(1+) sodium salt |
| 42 | 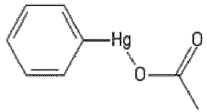 | Phenylmercuric acetate | acetyloxy(phenyl)mercury |
| 43 | 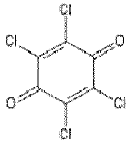 | Chloranil | 2,3,5,6-tetrachlorocyclohexa-2,5-diene-1,4-dione |
| 44 | 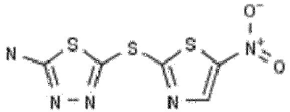 | SU3327 | 5-(5-Nitrothiazol-2-ylsulfanyl)-1,3,4-thiadiazol-2-amine |
| 45 | 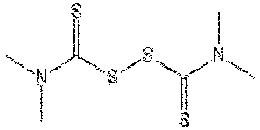 | Thiram | dimethylcarbamothioylsulfanyl *N ,N*-dimethylcarbamodithioate |
| 46 | 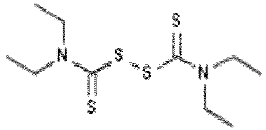 | Disulfiram | Tetraethylthioperoxydicarbonic diamide |
| 47 | 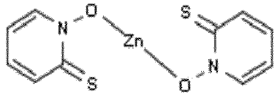 | Pyrithione Zinc | Bis[1-hydroxy-2(1H)-pyiridinethionato]zinc |
| 48 | 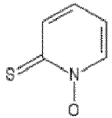 | Pyrithione | 1-hydroxypyridine-2-thione |
| 49 | 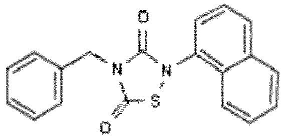 | Tideglusib | 4-Benzyl-2-(1-naphthyl)-1,2,4-thiadiazolidine-3,5-dione |
| 50 | 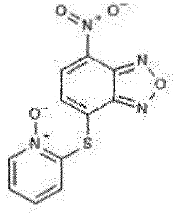 | NSC228155 | 4-Nitro-1-[(1-oxidopyridin-2-yl)sulfanyl]-2,1,3-benzoxadiazole |
| 51 | 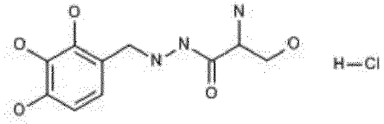 | Benserazide Hydrochloride | 2-Amino-3-hydroxy-N'-[(2,3,4-trihydroxyphenyl)methyl]propan ehydrazide hydrogen chloride |
| 52 | 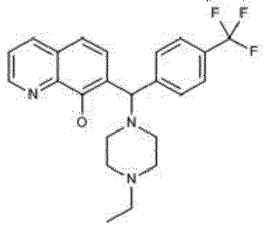 | ML311 | 7-[(4-Ethylpiperazin-1-yl)[4-(trifluoromethyl)phenyl]methyl]q uinolin-8-ol |
| 53 | 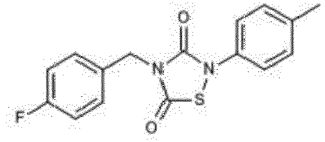 | CCG 50014 | 4-(4-Fluorobenzyl)-2-(4-methylphenyl)-1,2,4-thiadiazolidine-3,5-dione |
| 54 | 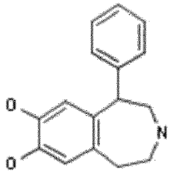 | SKF-38393 | 2,3,4,5,-Tetrahydro-7,8-dihydroxy-1-phenyl-1H-3-benzazepine |
| 55 | 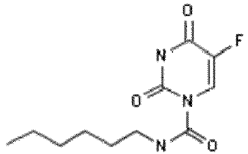 | Carmofur | 5-Fluoro-N-hexyl-2,4-dioxo-1,2,3,4-tetrahydropyrimidine-1-carboxamide |
| 56 | 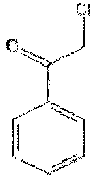 | Phenacyl chloride | 2-chloro-1-phenylethanone |
| 57 | 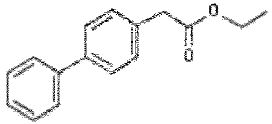 | Felbinac ethyl | [1,1'-Biphenyl]-4-acetic acid ethyl ester |
| 58 | 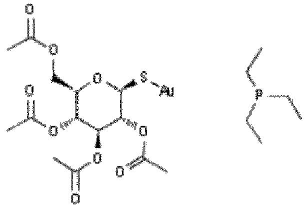 | Auranofin | (2,3,4,6-Tetra-O-acetyl-1-thio-beta-D-gl ucopyra nosato-S)(triethylphosphine)gold |
| 59 | 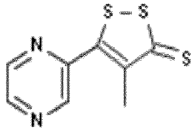 | Oltipraz | 4-Methyl-5-(2-pyrazinyl)-3H-1,2-dithiole-3-thione |
| 60 | 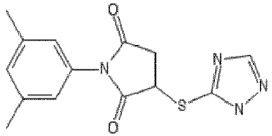 | WAY-308264 | 1-(3,5-dimethylphenyl)-3-(1H-1,2,4-triazol-5-ylsulfanyl)pyrrolidine-2,5-dione |
| 61 | 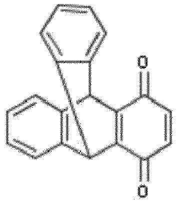 | INCA-6 | Pentacyclo[6.6.6.0(2,7).0(9,14).0( 15,20)]icosa-2(7),4,9,11,13,15,17,19-octaene-3,6-dione |
| 62 | 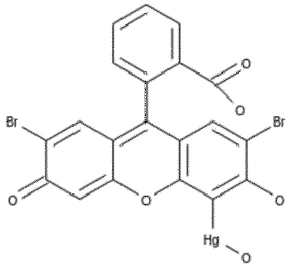 | Spectrum_00051 0 | [2,7-dibromo-9-(2-carboxyphenyl)-3-hydroxy-6-oxoxanthen-4-yl]mercury;hydrate |
| 63 | 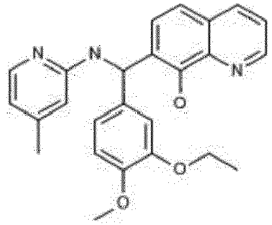 | HQ-415 | 7-[(3-Ethoxy-4-methoxyphenyl)[(4-methylpyridin-2-yl)amino]methyl]quinolin-8-ol |
| 64 | 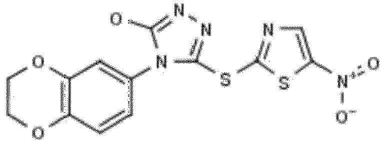 | BI-78D3 | 4-(2,3-Dihydro-1,4-benzodioxin-6-yl)-5-(5-nitrothiazol-2-ylsulfanyl)-4H-1,2,4-triazol-3-ol |
| 65 | 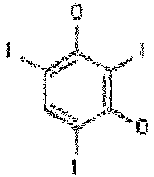 | Riodoxol | 2,4,6-Triiodoresorcine |
| 66 | 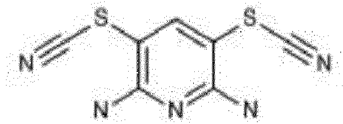 | PR-619 | 2,6-Diaminopyridine-3,5-diyl bis(thiocyanate) |
| 67 | 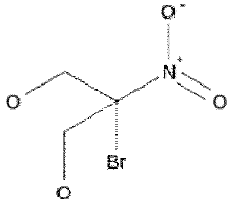 | Bronopol | 2-bromo-2-nitropropane-1,3-diol |
| 68 | 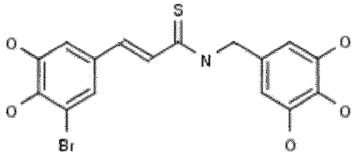 | NT157 | 3-(3-Bromo-4,5-dihydroxyphenyl)-N-(3,4,5-trihydroxybenzyl)-2-propenethioamide |
| 69 | 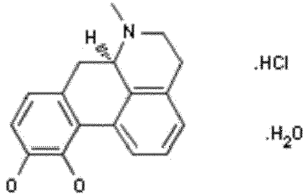 | Apomorphine hydrochloride | (R)-6-Methyl-5,6,6a,7-tetrahydro-4H-dibenzo[de,g]quinoline-10,11-diol hydrochloride hemihydrate |
| 70 | 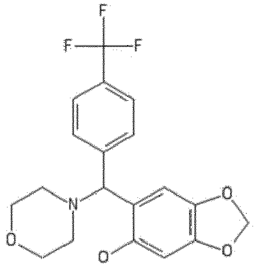 | PD120911 | 6-[morpholin-4-yl-[4-(trifluoromethyl)phenyl]methyl]-1,3-benzodioxol-5-ol |
| 71 | 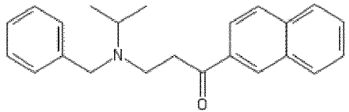 | ZM-39923 | 3-[benzyl(propan-2-yl)amino]-1-naphthalen-2-ylpropan-1-one |
| 72 | 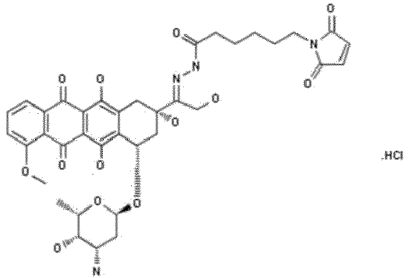 | Aldoxorubicin | N'-[1-[4(S)-(3-Amino-2,3,6-trideoxy-alpha-L-lyxo-hexopyranosyloxy)-2(S),5,12-trihydroxy-7-methoxy-6,11-dioxo-1,2,3,4,6,11-hexahydronaphthacen-2-yl]-2-hydroxyethylidene]-6-(2,5-dioxo-2,5-dihydro-1H-pyrrol-1-yl)hexanohydrazide hydrochloride |
| 73 | 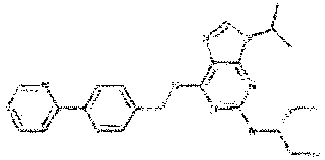 | CR8-(R) | (2R)-2-[[9-propan-2-yl-6-[(4-pyridin-2-ylphenyl)methylamino]purin-2-yl]amino]butan-1-ol |
| 74 | 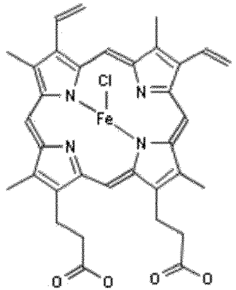 | Hemin | - (SP-5-13)-Chloro[3,8,13,17-tetramethyl-7,12-divinyl-21H,23H-porphine-2,18-dipropanoato(4-)-kappa N21,kappaN22,kappaN23,k appaN24]dihydrogenoferrate(2-) |
| 75 | 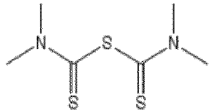 | Tetramethylthiur am monosulfide | dimethylcarbamothioyl N,N-dimethylcarbamodithioate |
| 76 | 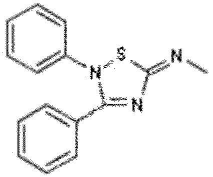 | SCH-202676 | N-(2,3-Diphenyl-2,5-dihydro-1,2,4-thiadiazol-5-ylidene)-N-methylamine |
| 77 | 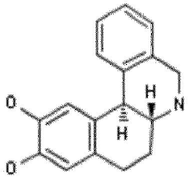 | Dihydrexidine | trans-10,11-Dihydroxy-5,6,6a,7,8,12b-hexahydrobenzo[a]phenanthridi ne |
| 78 | 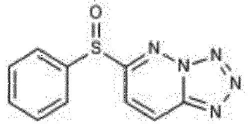 | Ro-106-9920 | 6-(Phenylsulfinyl)tetrazolo[1,5-b]pyridazine |
| 79 | 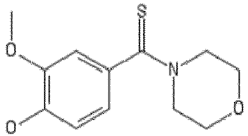 | Vanitiolide | (4-hydroxy-3-methoxyphenyl)-orpholin-4-ylmethanethione |
| 80 | 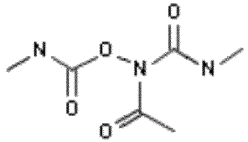 | Caracemide | N-[(Methylamino)carbonyl]-N-[[(methylamino)carbonyl]oxy]ace tamide |
| 81 | 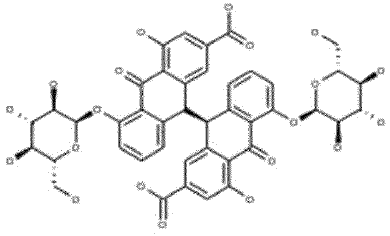 | Sennoside A | (9R)-9-[(9R)-2-carboxy-4-hydroxy-10-oxo-5-[(2S,3R,4S,5S,6R)-3,4,5-trihydroxy-6-(hydroxymethyl)oxan-2-yl]oxy-9H-anthracen-9-yl]-4-hydroxy-10-oxo-5-[(2S,3R,4S,5S,6R)-3,4,5-trihydroxy-6-(hydroxymethyl)oxan-2-yl]oxy-9H-anthracene-2-carboxylic acid |
| 82 | 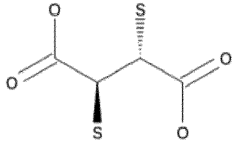 | Dimercaptosucci nic-acid | 2,3-bis(sulfanyl)butanedioic acid |
| 83 | 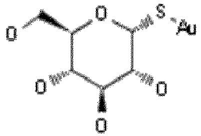 | Aurothioglucose | 1-Aurothioglucose |
| 84 | 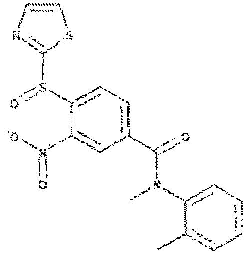 | SB-268262 | N-methyl-N-(2-methylphenyl)-3-nitro-4-(1,3-thiazol-2-ylsulfinyl)benzamide |
| 85 | 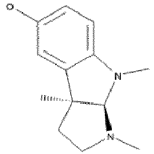 | eseroline-(-) | (3aS,8bR)-3,4,8b-trimethyl-2,3a-dihydro-1H-pyrrolo[2,3-b] indol-7-ol |
| 86 | 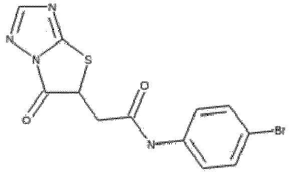 | PD096194 | N-(4-bromophenyl)-2-(6-oxo-[1,3]thiazolo[3,2-b][1,2,4]triazol-5-yl)acetamide |
| 87 | 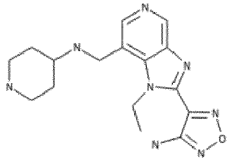 | SB-747651A | 4-[1-ethyl-1-[(piperidin-4-ylamino)methyl]imidazo[4,5-c]pyridin-2-yl]-1,2,5-oxadiazol-3-amine |
| 88 | 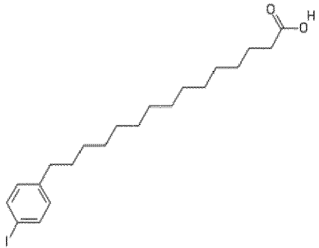 | omega-(4-lodophenyl)pent adecanoic acid | 15-(4-iodophenyl)pentadecanoic acid |
| 89 | 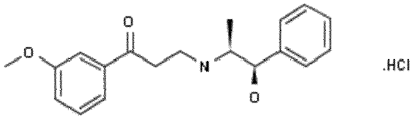 | oxyfedrine | 3-[2(R)-Hydroxy-1(S)-methyl-2-phenylethylamino]-1-(3-methoxyphenyl)propan-1-one hydrochloride |
| 90 | 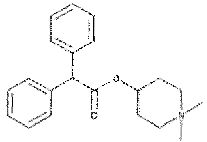 | 4-DAMP | (1,1-dimethylpiperidin-1-ium-4-yl) 2,2-diphenylacetate;iodide |
| 91 | 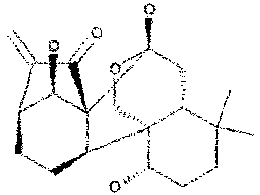 | Oridonin (Isodonol) | (1S,5S,8R,9S,10S,11R,15S,18R)-9,10,15,18-tetrahydroxy-12,12-dimethyl-6-methylidene-17-oxapentacyclo[7.6.2.15,8.01,11.0 2,8]octadecan-7-one |

and pharmaceutically acceptable salts thereof.

The identification in a subject of a SARS-Cov-2 infection is usually based on the analysis of the presence of viral RNA in biological material from the subject, preferably specimens from upper and lower respeiractory tract, such as nasopharyngeal/oropharyngeal swabs, nasal aspirate, nasal wash or saliva, sputum, tracheal aspirate or bronchoalveolar lavage. The subject to be treated may have one or more symptoms of COVID-19 or be asymptomatic.

As used herein the term "pharmaceutically acceptable salts" of the compounds above refers to addition salts of these compounds with acids or bases that form non-toxic acid anions or cations.

Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others.

The salts may be formed by conventional means, such as by reacting the free form of the compound with one or more equivalents of the appropriate acid or base in a solvent or medium in which the salt is insoluble, such as for example water or ethanol, which is removed under vacuum or by freeze drying.

The compounds above may be in an anhydrous or a hydrate form.

The present invention also includes prodrugs of the compounds above.

As used herein, the term "prodrug" refers to a biologically inactive compound which can be metabolized in the body to produce a compound listed above. An example, without limitation, of prodrugs are ester of the compounds according to the invention, that facilitate transmittal across a cell membrane where water solubility is not beneficial, but then are metabolically hydrolysed once inside the cell where water solubility is beneficial.

The compound according to the first object of the invention is administered in form of a pharmaceutical composition formed by admixture of the compound with one or more pharmaceutically acceptable excipients.

Thus, a second object of the invention is a pharmaceutical composition comprising a compound selected from the compounds above and pharmaceutically acceptable salts thereof, and at least one inert pharmaceutically acceptable excipient, for use in the treatment of a SARS-Cov-2 infection in a subject.

The route of administration of the compound or pharmaceutical composition of the present invention depends on the specific compound used or contained in the pharmaceutical composition and is in accordance with known methods for administration, which can be, for example inhalation, injection or infusion by intravenous, parenterally, topically, rectally, nasally, buccally, vaginally or via an implanted reservoir, or by sustained release systems. The term parenteral as used herein includes intravenous, intraperitoneal, intracerebral, intrathecal, intracranial, intramuscular, intraarticular, intrasynovial, intrasternal, intraocular, intraarterial, subcutaneous, and intracutaneous or intralesional injection or infusion techniques.

In one preferred embodiment, the invention provides for administration of the compound or the pharmaceutical composition of the invention such that a sufficient concentration of the compound is reached in the respiratory tract.

Preferably, this is obtained by direct administration of the compound or pharmaceutical composition of the present invention to the respiratory tract. The direct administration to the respiratory tract may be the sole route of administration of the compound or composition or may be combined with administration of the compaounsd or composition via other systemic routes.

For direct administration of the compound or composition of the invention, a wide variety of devices that are designed for the direct delivery of pharmaceutical compositions and therapeutic formulations to the respiratory tract, may be used. In one aspect of the present invention, the compound or pharmaceutical composition of the present invention is administered in aerosolized or inhaled form.

The pharmaceutical composition of the present invention for direct administration to the respiratory tract as described above, can be in form of an aerosol formulation, as a dry powder or a solution or suspension with a diluent.

Preferably, the pharmaceutically acceptable excipient includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions. Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, and salts for regulating osmotic pressure, emulsifiers, sweeteners, colorants, flavourings and the like.

The amount of said compound in the pharmaceutical composition of the present invention is the the amount usually employed for other indications of the specific compound.

The amount can vary over a wide range depending on known factors, for example, the molecule used, the severity of the disease, the patient's body weight, the dosage form, the chosen route of administration, and the number of administrations per day. However, a person skilled in the art can determine the optimum amount in easily and routinely manner.

Also specific dosage and treatment regimens for any particular patient will depend upon a variety of factors, including for example the activity and half life of the specific compound employed, the age, body weight, general health status, sex, diet, severity and course of the disease.

The dosage forms of the pharmaceutical composition of the present invention can be prepared by techniques that are familiar to a pharmaceutical chemist, and comprise mixing, granulation, compression, dissolution, sterilization and the like.

A third object of the invention is a method of treating a SARS-Cov-2 infection in a subject, comprising administering to said subject a compound selected from the compounds above and pharmaceutically acceptable salts therof.

The invention will be further described in the following examples, which do not limit the scope of the invention described in the claims.

### EXPERIMENTAL PART

### 1. Computer-Aided Drug Design

The sequence and crystal structure of the 3C-like protease P of SARS-CoV-2, was obtained from the Protein Data Bank with the code 6LU7 (https://www.rcsb.org/structure/6lu7).

The EXSCALATE platform, the most powerful computing resources currently based in Europe to empower smart in-silico drug design (co-funded by the H2020-FET-HPC ANTAREX project), was exploited to perform molecular dynamics (MD) and docking simulations, with the final aim to select and make available molecules active against the 3C-like protease of SARS-CoV-2. Molecular dynamics simulations on the 3C-like protease of SARS-CoV-2 were performed to explore the conformational space of the active site of the protease, and select several protein conformations particularly suitable for docking simulations.

In the first step, MD simulations were carried out on the structure of the 3C-like protease of SARS-CoV-2, prepared ad hoc in order to optimize the 3D structure from a chemical and conformational point of view. The structure was firstly subjected to a cycle of energy minimization by steepest descent methods to eliminate all initial steric clashes and obtain a pre-equilibrated model to start from. Then a 100 ps restrained MD simulation (typically 1000 KJ/mole force constant) was performed on the solvent atoms to equilibrate water molecules keeping the solute restrained. Finally, a production run was performed to generate a 1microsecond trajectory with a total of 20.000 collected. Post HPC-run analysis of the results was performed.

In a second step, High Performance Computing (HPC) simulation was conducted to virtual screen against the the 3C-like protease of SARS-CoV-2, the Safe in Man (SIM) library, containing commercialized and under development drugs, already proved safe in man (> 10,000 drugs), and the Fraunhofer's BROAD Repurposing Library, containing 5400 marketed drugs and clinical stage compounds and molecules with known mode of action. Duplicates between the libraries were removed before ligand preparation, where all compounds were converted to 3D and prepared with Schrödinger's LigPrep tool. This process generated multiple states for stereoisomers, tautomers, ring conformations (1 stable ring conformer by default) and protonation states. In particular, another Schrödinger package, Epik, was used to assign tautomers and protonation states that would be dominant at a selected pH range (pH=7±1). Ambiguous chiral centers were enumerated, allowing a maximum of 32 isomers to be produced from each input structure. Then, an energy minimization was performed with the OPLS3 force.

The simulation was performed using LiGen^{™} (Ligand Generator), the de novo structure based virtual screening software, designed and developed to run on HPC architectures, which represent the most relevant tool of the EXSCALATE platform. LiGenTM is formed by a set of tools that can be combined in a user-defined manner to generate project centric workflows. In particular, LiGenDock is a docking module using LiGenScore to compute the scoring function and the LiGenPass and LiGenPocket modules to obtain the 3D structure of the binding site. Both the docking algorithm implemented in LiGen, the pharmacophoric docking (LiGenPh4) and the geometrical docking (LiGenGeodock), as well as the different scoring functions calculated, were used in this study to explore different protocols of Virtual Screening (VS) and select the best one in terms of performance. The performances of the tested VS strategy were assessed by evaluating the capacity to correctly rank a set of active compounds. In particular, to this purpose, 190 known inhibitors of SARS-CoV-1 3C-like protease were included in the screened database and are considered as actives in the docking studies representing the training set in the optimization/validation analyses.

When tautomers were involved, only the one, with the best docking score, was chosen. The docking score values that predict the binding affinity of the molecules in the protein binding site, are reported (the higher, the better). Table 2 reports the results obtained for the best scored molecules, selected for further validation of the 3C-like protease of SARS-CoV-2 activity in in vitro experiments.

**Table 2**

| **N.** | **Current Name** | **Docking Score** |
|---|---|---|
| 1 | Baicalein | 5.86 |
| 2 | Dihydromyricetin | 5.73 |
| 3 | Myricetin | 5.63 |
| 4 | Apigenin | 5.79 |
| 5 | Myricitrin | 6.44 |
| 6 | (-)-Epigallocatechin Gallate | 6.30 |
| 7 | Lansoprazole | 5.77 |
| 8 | Rabeprazole (sodium) | 5.36 |
| 9 | Tenatoprazole | 5.43 |
| 10 | Ebselen | 5.29 |
| 11 | MLS-0315771 | 5.82 |
| 12 | PBIT | 5.80 |
| 13 | ML345 Analog | 5.92 |
| 14 | PD086290 | 5.52 |
| 15 | RI-1 | 5.88 |
| 16 | PD119792 | 5.83 |
| 17 | Bay-11-7082 | 5.10 |
| 18 | Bay-11-7085 | 5.42 |
| 19 | TZDZ-8 | 5.12 |
| 20 | CL-17107 | 5.18 |
| 21 | Tosyl Phenanyl Chloromethyl Ketone | 5.80 |
| 22 | Nordihydroguaiaretic acid | 5.94 |
| 23 | Captan | 5.42 |
| 24 | Benzo[d]thiazole-2(3H)-thione | 5.70 |
| 25 | Benzothiazyl disulfide | 5.72 |
| 26 | CBS, N-Cyclohexyl-2-benzothiazolesulfenamide | 5.65 |
| 27 | N-oxydiethylenebenzothiazole-2-sulfenamide | 5.18 |
| 28 | PX-12 | 5.98 |
| 29 | Carboxypyridine-disulfide | 5.63 |
| 30 | IPA-3 | 6.18 |
| 32 | Bonaphthone | 5.51 |
| 33 | JAK3-inhibitor-V | 5.54 |
| 34 | PD119507 | 5.98 |
| 35 | Pyrrolidine-dithiocarbamate | 5.43 |
| 36 | MIRA-1 | 5.73 |
| 37 | Hematoxylin (Hydroxybrazilin) | 5.94 |
| 38 | TAS-103 (dihydrochloride) | 6.19 |
| 39 | Calpeptin | 5.92 |
| 40 | Z-DEVD-FMK | 5.90 |
| 43 | Chloranil | 5.65 |
| 44 | SU3327 | 5.20 |
| 45 | Thiram | 5.36 |
| 46 | Disulfiram | 5.96 |
| 48 | Pyrithione | 5.22 |
| 49 | Tideglusib | 6.20 |
| 50 | NSC228155 | 5.48 |
| 51 | Benserazide Hydrochloride | 5.93 |
| 52 | ML311 | 6.72 |
| 53 | CCG 50014 | 5.99 |
| 54 | SKF-38393 | 5.77 |
| 55 | Carmofur | 5.34 |
| 56 | Phenacyl chloride | 5.84 |
| 57 | Felbinac ethyl | 5.62 |
| 59 | Oltipraz | 5.10 |
| 60 | WAY-308264 | 5.75 |
| 61 | INCA-6 | 5.66 |
| 62 | Spectrum_000510 | 6.61 |
| 63 | HQ-415 | 6.61 |
| 64 | BI-78D3 | 5.77 |
| 65 | Riodoxol | 5.14 |
| 66 | PR-619 | 5.83 |
| 67 | Bronopol | 5.61 |
| 68 | NT157 | 5.91 |
| 69 | Apomorphine hydrochloride | 5.83 |
| 70 | PD120911 | 6.18 |
| 71 | ZM-39923 | 6.40 |
| 72 | Aldoxorubicin | 5.94 |
| 73 | CR8-(R) | 6.64 |
| 74 | Hemin | 7.19 |
| 75 | Tetramethylthiuram monosulfide | 5.32 |
| 76 | SCH-202676 | 5.82 |
| 77 | Dihydrexidine | 5.71 |
| 78 | Ro-106-9920 | 5.39 |
| 79 | Vanitiolide | 5.22 |
| 80 | Caracemide | 5.57 |
| 82 | Dimercaptosuccinic-acid | 5.51 |
| 84 | SB-268262 | 6.29 |
| 85 | eseroline-(-) | 5.30 |
| 86 | PD096194 | 5.73 |
| 87 | SB-747651A | 5.86 |
| 88 | omega-(4-lodophenyl)pentadecanoic acid | 5.36 |
| 89 | oxyfedrine | 5.65 |
| 90 | 4-DAMP | 5.91 |
| 91 | Oridonin (Isodonol) | 6.15 |

### 2. In vitro screening of activity against SARS-CoV-2: 3CLpro biochemical assay

Compound repurposing is an important strategy for the identification of effective treatment options against SARS-CoV-2 infection and COVID-19 disease. As a single-stranded positive-sense RNA virus, SARS-CoV-2 uses cellular translation machinery directly after infection of the cell to produce viral polyproteins pp1a and pp1ab. The generation of individual viral proteins involves proteolytic cleavage by 3CL-Pro and PLpro (PMID: 32292689).

In this regard, SARS-CoV-2 main protease (3CL-Pro), also termed M-Pro, is an attractive drug target as it plays a central role in viral replication. To this aim we screened 8702 compound for the ability of inhibiting 3CLpro protease activity on a FRET based assay.

The detection of enzymatic activity of the SARS-CoV-2 3CL-Pro was performed under conditions similar to those reported by Zhang et al (PMID: 32198291) and adjusted as follow. Enzymatic activity was measured by a Förster resonance energy transfer (FRET), using the dual-labelled substrate, DABCYL-KTSAVLQ↓SGFRKM-EDANS (Bachem #4045664) containing a protease specific cleavage site after the Gln. In the intact peptide, EDANS fluorescence is quenched by the DABCYL group. Following enzymatic cleavage, generation of the fluorescent product was monitored (Ex/Em= 340/460nm), (EnVision, Perkin Elmer).

In the primary screen, test compounds (stock at 10 mM in 100 % DMSO), positive (zinc pyrithione (medchemexpress, #HY-B0572) 10 mM in 100 % DMSO) and negative (100 % DMSO) controls, were transferred to 384-well assay microplates by acoustic dispensing (Echo, Labcyte). 5 µl of SARS-CoV-2 3CL-Pro stock (120 nM) in assay buffer were added to compound plates and incubated for 60 min at 37 °C. This pre-incubation step facilitated the identification of slowly binding putative cysteine-reactive inhibitors. After addition of 5 µl substrate (30 µM in assay buffer), the final concentrations were: 15 µM substrate; 60 nM SARS-CoV-2 3CL-Pro; and 0.2 % DMSO in a total volume of 10 µL/well. The fluorescence signal was then measured at 15 min and inhibition (%) calculated relative to controls (Envision, PerkinElmer). Results were normalized to the 100 % (positive control) and 0 % (negative control) inhibition. To flag possible optical interference effects, primary assay plates were also read 60 min after substrate addition, when the reaction was complete. In this experimental set, some compounds used as positive controls slow down or lose their inhibition activity in the presence of DTT. So to, account for any DTT dependent effects, primary screening was performed without DTT in the assay buffer.

Primary screening to assess 3CLpro inhibition was performed at a unique test compound concentration attested at 20 µM. The resulting compounds were profiled in dose response achieved with serial dilution at 8 different concentration points following a log dilution (starting concentration 0.033 µM) in absence of DTT.

We finally selected 86 compounds with IC50 values below 3 µM, and 5 compounds with IC50 between 3 and 5, for further investigation.

IC50 (µM): indicates the half maximal concentration needed to reach the maximal value of enzyme inhibition. It was determined from dose -response curves obtained testing the compounds antiviral effect at 8 different concentrations.

Inhibition (%): This parameter is strictly linked to fluorescence signal registered during the assay. Given that positive and negative control fluorescence is considered 100% and 0%, respectively; the fluorescence registered in compounds wells is reported to this scale. Interestingly, almost all the selected compounds have an inhibition higher than 80%.

The results obtained for the compounds tested are shown in Table 3 below.

**Table 3**

| **N.** | **Current Name** | **IC50 (µM)** | **3CL-Pro Inhibition [%] Primary Screen (20 µM)** |
|---|---|---|---|
| 1 | Baicalein | 0.02 | 97.8 |
| 2 | Dihydromyricetin | 0.18 | 99.7 |
| 3 | Myricetin | 0.22 | 97.4 |
| 4 | Apigenin | 3.02 | 94.5 |
| 5 | Myricitrin | 3.66 | 90.56 |
| 6 | (-)-Epigallocatechin Gallate | 0.72 | 95.3 |
| 7 | Lansoprazole | 1.6 | 101.12 |
| 8 | Rabeprazole (sodium) | 0.74 | 94.3 |
| 9 | Tenatoprazole | 0.97 | 69.1 |
| 10 | Ebselen | 0.03 | 100 |
| 11 | MLS-0315771 | 0.02 | 100.74 |
| 12 | PBIT | 0.02 | 100.77 |
| 13 | ML345 Analog | 0.02 | 100.94 |
| 14 | PD086290 | 0.02 | 100.12 |
| 15 | RI-1 | 1.14 | 99.2 |
| 16 | PD119792 | 1.23 | 100 |
| 17 | Bay-11-7082 | 0.82 | 95.7 |
| 18 | Bay-11-7085 | 0.84 | 100 |
| 19 | TZDZ-8 | 0.14 | 101 |
| 20 | CL-17107 | 0.39 | 101 |
| 21 | Tosyl Phenanyl Chloromethyl Ketone | 1.37 | 99.7 |
| 22 | Nordihydroguaiaretic acid | 2.59 | 98.5 |
| 23 | Captan | 0.67 | 99.8 |
| 24 | Benzo[d]thiazole-2(3H)-thione | 1.74 | 94 |
| 25 | Benzothiazyl disulfide | 0.16 | 83.5 |
| 26 | CBS, N-Cyclohexyl-2-benzothiazolesulfenamide | 0.77 | 70.1 |
| 27 | N-oxydiethylenebenzothiazole-2-sulfenamide | 5.05 | 98.98 |
| 28 | PX-12 | 0.14 | 100 |
| 29 | Carboxypyridine-disulfide | 0.24 | 100 |
| 30 | IPA-3 | 0.51 | 91.3 |
| 31 | Silver-sulfadiazine | 0.75 | 99.7 |
| 32 | Bonaphthone | 0.04 | 102 |
| 33 | JAK3-inhibitor-V | 1.24 | 94.5 |
| 34 | PD119507 | 2.05 | 99.3 |
| 35 | Pyrrolidine-dithiocarbamate | 0.21 | 101 |
| 36 | MIRA-1 | 0.29 | 100 |
| 37 | Hematoxylin (Hydroxybrazilin) | 0.22 | 99.6 |
| 38 | TAS-103 (dihydrochloride) | 2.99 | 96.1 |
| 39 | Calpeptin | 4.74 | 81.01 |
| 40 | Z-DEVD-FMK | 0.01 | 100 |
| 41 | Thimerosal | 0.01 | 100 |
| 42 | Phenylmercuric acetate | 0.01 | 100 |
| 43 | Chloranil | 0.02 | 101 |
| 44 | SU3327 | 0.03 | 99.9 |
| 45 | Thiram | 0.05 | 99.9 |
| 46 | Disulfiram | 0.22 | 99.7 |
| 47 | Pyrithione Zinc | 2.46 | 99.7 |
| 48 | Pyrithione | 0.05 | 101 |
| 49 | Tideglusib | 0.08 | 101 |
| 50 | NSC228155 | 0.1 | 102 |
| 51 | Benserazide Hydrochloride | 0.14 | 94.3 |
| 52 | ML311 | 0.15 | 100 |
| 53 | CCG 50014 | 0.15 | 101 |
| 54 | SKF-38393 | 2.63 | 99.9 |
| 55 | Carmofur | 0.16 | 101 |
| 56 | Phenacyl chloride | 0.19 | 99.9 |
| 57 | Felbinac ethyl | 0.2 | 96.2 |
| 58 | Auranofin | 0.21 | 100 |
| 59 | Oltipraz | 0.21 | 101 |
| 60 | WAY-308264 | 3.17 | 89.41 |
| 61 | INCA-6 | 0.24 | 99.2 |
| 62 | Spectrum_000510 | 0.24 | 101 |
| 63 | HQ-415 | 0.27 | 98.9 |
| 64 | BI-78D3 | 0.3 | 96.4 |
| 65 | Riodoxol | 0.33 | 99.7 |
| 66 | PR-619 | 0.41 | 97.6 |
| 67 | Bronopol | 0.42 | 99.9 |
| 68 | NT157 | 0.48 | 79.4 |
| 69 | Apomorphine hydrochloride | 0.52 | 79 |
| 70 | PD120911 | 0.54 | 81.4 |
| 71 | ZM-39923 | 0.58 | 75.7 |
| 72 | Aldoxorubicin | 0.58 | 101 |
| 73 | CR8-(R) | 0.84 | 99.5 |
| 74 | Hemin | 0.88 | 96.6 |
| 75 | Tetramethylthiuram monosulfide | 0.94 | 99.9 |
| 76 | SCH-202676 | 0.98 | 99.8 |
| 77 | Dihydrexidine | 1.05 | 98.3 |
| 78 | Ro-106-9920 | 1.12 | 100 |
| 79 | Vanitiolide | 1.32 | 100 |
| 80 | Caracemide | 1.33 | 96.4 |
| 81 | Sennoside A | 1.59 | 103 |
| 82 | Dimercaptosuccinic-acid | 1.72 | 98.9 |
| 83 | Aurothioglucose | 1.74 | 97.3 |
| 84 | SB-268262 | 1.87 | 96.7 |
| 85 | eseroline-(-) | 1.99 | 91.5 |
| 86 | PD096194 | 2.04 | 101 |
| 87 | SB-747651A | 2.07 | 96.2 |
| 88 | omega-(4-lodophenyl)pentadecanoic acid | 2.25 | 95 |
| 89 | oxyfedrine | 2.35 | 90.8 |
| 90 | 4-DAMP | 2.36 | 97.8 |
| 91 | Oridonin (Isodonol) | 2.57 | 98.2 |

## Claims

1. A compound for use in the treatment of a SARS-Cov-2 infection in a subject, selected from:
a compound of formula (I) 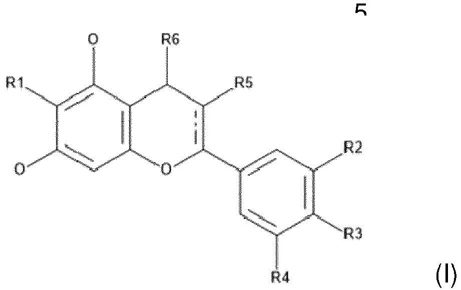 wherein
R1, R2, R3 and R4 are independently selected from OH and H,
R5 is selected from the group consisting of OH, H, alpha-L-rhamnopyranosidyl and 3,4,5-trihydroxybenzoate
R6 is selected from H and carbonyl,
with the proviso that at least one of the following condition is satisfied:
R1 is OH, or
R2, R3, R4 are OH or
R3 is OH and the fused rings are a chromone;
a compound of formula (II) 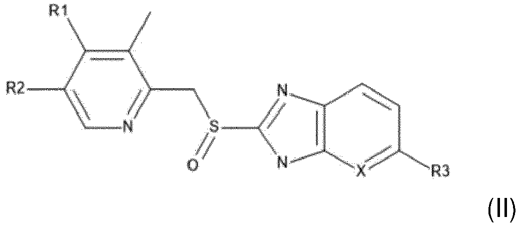 wherein
R1 is selected from the group consisting of OCH₂CF₃, OCH₂CH₂CH₂OCH₃ and OCH₃,
R2 is selected from H and CH₃,
R3 is selected from H and OCH₃,
X is selected from N and C;
a compound of formula (III) 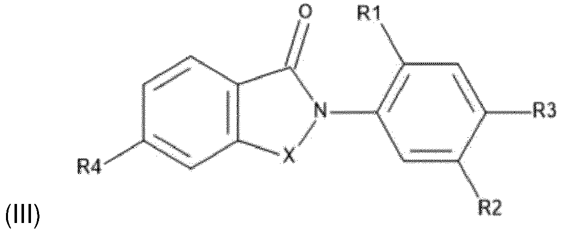 wherein
R1 is selected from H and CH₃,
R2 is selected from the group consisting of H, CH₃ and SO₂N(CH₃)₂,
R3 is selected from H and CH₃,
R4 is selected from H and F,
X is selected from S and Se;
a compound of formula (IV) 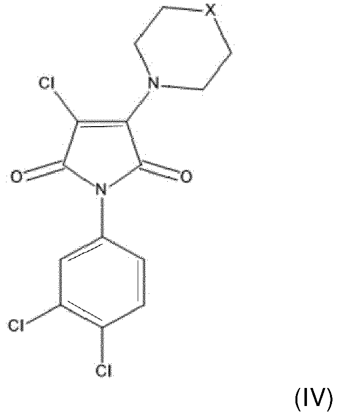 wherein
X is selected from O and NCH₃;
a compound of formula (V) 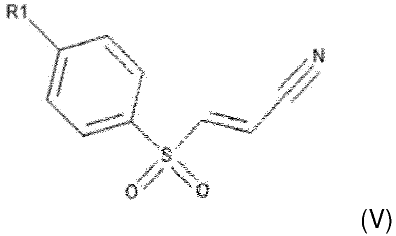 wherein
R1 is selected from CH₃ and C(CH₃)₃;
a compound of formula (VI) 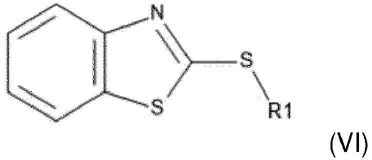 wherein
R1 is selected from the group consisting of H, N-Cyclohexyl, S-benzothiazol-2-yl and morpholin-4-yl;
a compound of formula (VII) 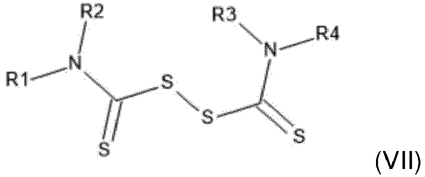 wherein R1, R2, R3 and R4 are independently selected from CH₃ and CH₂CH₃;
a compound of formula (VIII) 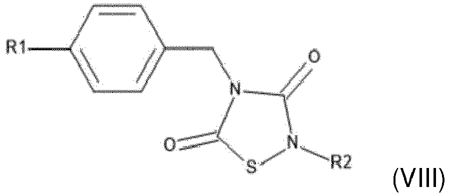 wherein
R1 is selected from H and F,
R2 is selected from 4-methylphenyl and 1-naphthalenyl;
a compound of formula (IX) 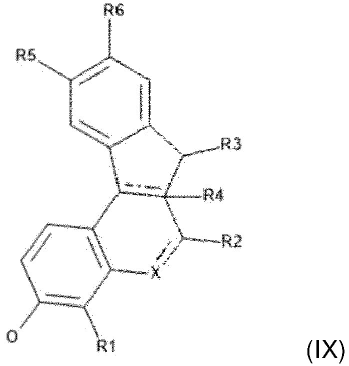 wherein
R1, R4, R5, R6 are independently selected from H and OH,
R2 is selected from H and NCH₂CH₂N(CH₃)₂,
R3 is selected from H and carbonyl,
X is selected from N and O;
the compounds listed in the table below:
| **Current Name** | **Chemical Name** |
|---|---|
| TZDZ-8 | 4-Benzyl-2-methyl-1,2,4-thiadiazolidine-3,5-dione |
| CL-17107 | 5-benzyl-3H-1,3,4-thiadiazole-2-thione |
| Tosyl Phenanyl Chloromethyl Ketone | N-[(2S)-5-chloro-3,4-dioxo-1-phenylpentan-2-yl]-4-methylbenzenesulfonamide |
| Nordihydroguaiaretic acid | 4-[4-(3,4-dihydroxyphenyl)-2,3-dimethylbutyl]benzene-1,2-diol |
| Captan | 2-(trichloromethylsulfanyl)-3a,4,7,7a-tetrahydroisoindole-1,3-dione |
| PX-12 | (2-Imidazolyl)(1-methylpropyl)disulfide |
| Carboxypyridine-disulfide | 6-[(5-carboxypyridin-2-yl)disulfanyl]pyridine-3-carboxylic acid |
| IPA-3 | 1,1'-Disulfanediylbis(2-naphthol) |
| Silver-sulfadiazine | silver;(4-aminophenyl)sulfonyl-pyrimidin-2-ylazanide |
| Bonaphthone | 6-Bromonaphthalene-1,2-dione |
| JAK3-inhibitor-V | 1-naphthalen-2-ylprop-2-en-1-one |
| PD119507 | 5-aminoquinolin-8-ol |
| Pyrrolidine-dithiocarbamate | N-pyrrolidin-1-ylcarbamodithioate |
| MIRA-1 | Propionic acid 2,5-dioxo-2,5-dihydro-1H-pyrrol-1-ylmethyl ester |
| Calpeptin | N2-[(Benzyloxy)carbonyl]-N-[(2S)-1-oxohexan-2-yl]-L-leucinamide |
| Z-DEVD-FM K | N-(Benzyloxycarbonyl)-L-aspartyl-L-glutamyl-L-valyl-L-aspartylfluoromethane |
| Thimerosal | 2-Sulfidobenzoic acid ethylmercury(1+) sodium salt |
| Phenylmercuric acetate | acetyloxy(phenyl)mercury |
| Chloranil | 2,3,5,6-tetrachlorocyclohexa-2,5-diene-1,4-dione |
| SU3327 | 5-(5-Nitrothiazol-2-ylsulfanyl)-1,3,4-thiadiazol-2-amine |
| Pyrithione Zinc | Bis[1-hydroxy-2(1H)-pyiridinethionato]zinc |
| Pyrithione | 1-hydroxypyridine-2-thione |
| NSC228155 | 4-Nitro-7-[(1-oxidopyridin-2-yl)sulfanyl]-2,1,3-benzoxadiazole |
| Benserazide Hydrochloride | 2-Amino-3-hydroxy-N'-[(2,3,4-trihydroxyphenyl)methyl]propanehydrazide hydrogen chloride |
| ML311 | 7-[(4-Ethylpiperazin-1-yl)[4-(trifluoromethyl)phenyl]methyl]quinolin-8-ol |
| SKF-38393 | 2,3,4,5,-Tetrahydro-7,8-dihydroxy-1-phenyl-1H-3-benzazepine |
| Carmofur | 5-Fluoro-N-hexyl-2,4-dioxo-1,2,3,4-tetrahydropyrimidine-1-carboxamide |
| Phenacyl chloride | 2-chloro-1-phenylethanone |
| Felbinac ethyl | [1,1'-Biphenyl]-4-acetic acid ethyl ester |
| Auranofin | (2,3,4,6-Tetra-O-acetyl-1-thio-beta-D-glucopyranosato-S)(triethylphosphine)gold |
| Oltipraz | 4-Methyl-5-(2-pyrazinyl)-3H-1,2-dithiole-3-thione |
| WAY-308264 | 1-(3,5-dimethylphenyl)-3-(1H-1,2,4-triazol-5-ylsulfanyl)pyrrolidine-2,5-dione |
| INCA-6 | Pentacyclo[6.6.6.0(2,7).0(9,14).0(15,20)]icosa-2(7),4,9,11,13,15,17,19-octaene-3,6-dione |
| Spectrum_000510 | [2,7-dibromo-9-(2-carboxyphenyl)-3-hydroxy-6-oxoxanthen-4-yl]mercury;hydrate |
| HQ-415 | 7-[(3-Ethoxy-4-methoxyphenyl)[(4-methylpyridin-2-yl)amino]methyl]quinolin-8-ol |
| BI-78D3 | 4-(2,3-Dihydro-1,4-benzodioxin-6-yl)-5-(5-nitrothiazol-2-ylsulfanyl)-4H-1,2,4-triazol-3-ol |
| Riodoxol | 2,4,6-Triiodoresorcine |
| PR-619 | 2,6-Diaminopyridine-3,5-diyl bis(thiocyanate) |
| Bronopol | 2-bromo-2-nitropropane-1,3-diol |
| NT157 | 3-(3-Bromo-4,5-dihydroxyphenyl)-N-(3,4,5-trihydroxybenzyl)-2-propenethioamide |
| Apomorphine hydrochloride | (R)-6-Methyl-5,6,6a,7-tetrahydro-4H-dibenzo[de,g]quinoline-10,11-diol hydrochloride hemihydrate |
| PD120911 | 6-[morpholin-4-yl-[4-(trifluoromethyl)phenyl]methyl]-1,3-benzodioxol-5-ol |
| ZM-39923 | 3-[benzyl(propan-2-yl)amino]-1-naphthalen-2-ylpropan-1-one |
| Aldoxorubicin | N'-[1-[4(S)-(3-Amino-2,3,6-trideoxy-alpha-L-lyxo-hexopyranosyloxy)-2(S),5,12-trihydroxy-7-methoxy-6,11-dioxo-1,2,3,4,6,11-hexahydronaphthacen-2-yl]-2-hydroxyethylidene]-6-(2,5-dioxo-2,5-dihydro-1H-pyrrol-1-yl)hexanohydrazide hydrochloride |
| CR8-(R) | (2R)-2-[[9-propan-2-yl-6-[(4-pyridin-2-ylphenyl)methylamino]purin-2-yl]amino]butan-1-ol |
| Hemin | - (SP-5-13)-Chloro[3,8,13,17-tetramethyl-7,12-divinyl-21H,23H-porphine-2,18-dipropanoato(4-)-kappaN21,kappaN22,kappaN23,kappaN24]dihydrogenoferrate(2-) |
| Tetramethylthiuram monosulfide | dimethylcarbamothioyl N,N-dimethylcarbamodithioate |
| SCH-202676 | N-(2,3-Diphenyl-2,5-dihydro-1,2,4-thiadiazol-5-ylidene)-N-methylamine |
| Dihydrexidine | trans-10,11-Dihydroxy-5,6,6a,7,8,12b-hexahydrobenzo[a]phenanthridine |
| Ro-106-9920 | 6-(Phenylsulfinyl)tetrazolo[1,5-b]pyridazine |
| Vanitiolide | (4-hydroxy-3-methoxyphenyl)-morpholin-4-ylmethanethione |
| Caracemide | N-[(Methylamino)carbonyl]-N-[[(methylamino)carbonyl]oxy]acetamide |
| Sennoside A | (9R)-9-[(9R)-2-carboxy-4-hydroxy-10-oxo-5-[(2S,3R,4S,5S,6R)-3,4,5-trihydroxy-6-(hydroxymethyl)oxan-2-yl]oxy-9H-anthracen-9-yl]-4-hydroxy-10-oxo-5-[(2S,3R,4S,5S,6R)-3,4,5-trihydroxy-6-(hydroxymethyl)oxan-2-yl]oxy-9H-anthracene-2-carboxylic acid |
| Dimercaptosuccinic-acid | 2,3-bis(sulfanyl)butanedioic acid |
| Aurothioglucose | 1-Aurothioglucose |
| SB-268262 | N-methyl-N-(2-methylphenyl)-3-nitro-4-(1,3-thiazol-2-ylsulfinyl)benzamide |
| eseroline-(-) | (3aS,8bR)-3,4,8b-trimethyl-2,3a-dihydro-1H-pyrrolo[2,3-b]indol-7-ol |
| PD096194 | N-(4-bromophenyl)-2-(6-oxo-[1,3]thiazolo[3,2-b][1,2,4]triazol-5-yl)acetamide |
| SB-747651A | 4-[1-ethyl-7-[(piperidin-4-ylamino)methyl]imidazo[4,5-c]pyridin-2-yl]-1,2,5-oxadiazol-3-amine |
| omega-(4-lodophenyl)pentadecanoic acid | 15-(4-iodophenyl)pentadecanoic acid |
| oxyfedrine | 3-[2(R)-Hydroxy-1(S)-methyl-2-phenylethylamino]-1-(3-methoxyphenyl)propan-1-one hydrochloride |
| 4-DAMP | (1,1-dimethylpiperidin-1-ium-4-yl) 2, 2-di phenylacetate; iodide |
| Oridonin (Isodonol) | (1S,5S,8R,9S,10S,11R,15S,18R)-9,10,15,18-tetrahydroxy-12,12-dimethyl-6-methylidene-17-oxapentacyclo[7.6.2.15,8.01,11.02,8]octadecan-7-one |
and pharmaceutically acceptable salts thereof.

2. The compound for use according to claim 1, wherein
said compound of formula (I) is selected from the group consisting of 5,6,7-Trihydroxy-2-phenyl-4H-1-benzopyran-4-one, (+)-3(R),5,7-Trihydroxy-2(R)-(3,4,5-trihydroxyphenyl)-3,4-dihydro-2H-1-benzopyran-4-one, 3,5,7-Trihydroxy-2-(3,4,5-trihydroxyphenyl)-4H-1-benzopyran-4-one, 4',5,7-Trihydroxyflavone and 5,7-Dihydroxy-4-oxo-2-(3,4,5-trihydroxyphenyl)-4H-1-benzopyran-3-yl alpha-L-rhamnopyranoside, (-)-3,4,5-Trihydroxybenzoic acid 5,7-dihydroxy-2(R)-(3,4,5-trihydroxyphenyl)-3,4-dihydro-2H-1-benzopyran-3(R)-yl ester, and pharmaceutically acceptable salts thereof
or
said compound of formula (II) is selected from the group consisting of 2-([[3-Methyl-4-(2,2,2-trifluoroethoxy)pyridin-2-yl]methyl]sulfinyl)-1H-benzimidazole, 2-[4-(3-Methoxypropoxy)-3-methylpyridin-2-ylmethylsulfinyl]benzimidazol-1-ide sodium salt and (±)-5-Methoxy-2-(4-methoxy-3,5-dimethylpyridin-2-ylmethylsulfinyl)imidazo[4,5-b]pyridine, and pharmaceutically acceptable salts thereof
or
said compound of formula (III) is selected from the group consisting of 2-Phenyl-1,2-benzisoselenazol-3(2H)-one, 2-(2,5-Dimethylphenyl)-6-fluoro-1,2-benzothia zol-3(2H)-one, 2-(4-methylphenyl)-1,2-benzothiazol-3-one, N,N-dimethyl-3-(3-oxo-1,2-benzothiazol-2-yl)benzenesulfonamide and 2-phenyl-1,2-benzothiazol-3-one, and pharmaceutically acceptable salts thereof
or
said compound of formula (IV) is selected from 3-chloro-1-(3,4-dichlorophenyl)-4-morpholin-4-ylpyrrole-2,5-dione and 3-chloro-1-(3,4-dichlorophenyl)-4-(4-methylpiperazin-1-yl)pyrrole-2,5-dione, and pharmaceutically acceptable salts thereof
or
said compound of formula (V) is selected from 3-(4-Methylphenylsulfonyl)-2-propenenitrile and 3-(4-tert-Butylphenylsulfonyl)-2(E)-propenenitrile, and pharmaceutically acceptable salts thereof
or
said compound of formula (VI) is selected from the group consisting of 3H-1,3-benzothiazole-2-thione, N-(1,3-benzothiazol-2-ylsulfanyl)cyclohexanamine, 4-(1,3-benzothiazol-2-ylsulfanyl)morpholine and 2-(1,3-benzothiazol-2-yldisulfanyl)-1,3-benzothiazole, and pharmaceutically acceptable salts thereof
or
said compound of formula (VII) is selected from dimethylcarbamothioylsulfanyl N,N-dimethylcarbamodithioate and Tetraethylthioperoxydicarbonic diamide, and pharmaceutically acceptable salts thereof
or
said compound of formula (VIII) is selected from 4-Benzyl-2-(1-naphthyl)-1,2,4-thiadiazolidine-3,5-dione and 4-(4-Fluorobenzyl)-2-(4-methylphenyl)-1,2,4-thiadiazolidine-3,5-dione, and pharmaceutically acceptable salts thereof
or
said compound of formula (IX) is selected from (6aS,11bR)-7,11b-dihydro-6H-indeno[2,1-c]chromene-3,4,6a,9,10-pentol and 6-[2-(Dimethylamino)ethylamino]-3-hydroxy-7H-indeno[2,1-c]quinolin-7-one dihydrochloride, and pharmaceutically acceptable salts thereof.

3. The compound for use according to claim 1 or claim 2, selected from the compounds listed in the table below
| **Current Name** | **Chemical Name** |
|---|---|
| Baicalein | 5,6,7-Trihydroxy-2-phenyl-4H-1-benzopyran-4-one |
| Dihydromyricetin | (+)-3(R),5,7-Trihydroxy-2(R)-(3,4,5-trihydroxyphenyl)-3,4-dihydro-2H-1-benzopyran-4-one |
| Myricetin | 3,5,7-Trihydroxy-2-(3,4,5-trihydroxyphenyl)-4H-1-benzopyran-4-one |
| Apigenin | 4',5,7-Trihydroxyflavone |
| Myricitrin | 5,7-Dihydroxy-4-oxo-2-(3,4,5-trihydroxyphenyl)-4H-1-benzopyran-3-yl alpha-L-rhamnopyranoside |
| (-)-Epigallocatechin Gallate | (-)-3,4,5-Trihydroxybenzoic acid 5,7-dihydroxy-2(R)-(3,4,5-trihydroxyphenyl)-3,4-dihydro-2H-1-benzopyran-3(R)-yl ester |
| Lansoprazole | 2-([[3-Methyl-4-(2,2,2-trifluoroethoxy)pyridin-2-yl]methyl]sulfinyl)-1H-benzimidazole |
| Rabeprazole (sodium) | 2-[4-(3-Methoxypropoxy)-3-methylpyridin-2-ylmethylsulfinyl]benzimidazol-1-ide sodium salt |
| Tenatoprazole | (±)-5-Methoxy-2-(4-methoxy-3,5-dimethylpyridin-2-ylmethylsulfinyl)imidazo[4,5-b]pyridine |
| Ebselen | 2-Phenyl-1,2-benzisoselenazol-3(2H)-one |
| M LS-0315771 | 2-(2,5-Dimethylphenyl)-6-fluoro-1,2-benzothiazol-3(2H)-one |
| PBIT | 2-(4-methyl phenyl)-1,2-benzothiazol-3-one |
| ML345 Analog | N,N-dimethyl-3-(3-oxo-1,2-benzothiazol-2-yl)benzenesulfonamide |
| PD086290 | 2-phenyl-1,2-benzothiazol-3-one |
| RI-1 | 3-chloro-1-(3,4-dichlorophenyl)-4-morpholin-4-ylpyrrole-2,5-dione |
| PD119792 | 3-chloro-1-(3,4-dichlorophenyl)-4-(4-methylpiperazin-1-yl)pyrrole-2,5-dione |
| Bay-11-7082 | 3-(4-Methylphenylsulfonyl)-2-propenenitrile |
| Bay-11-7085 | 3-(4-tert-Butylphenylsulfonyl)-2(E)-propenenitrile |
| TZDZ-8 | 4-Benzyl-2-methyl-1,2,4-thiadiazolidine-3,5-dione |
| CL-17107 | 5-benzyl-3H-1,3,4-thiadiazole-2-thione |
| Tosyl Phenanyl Chloromethyl Ketone | N-[(2S)-5-chloro-3,4-dioxo-1-phenylpentan-2-yl]-4-methylbenzenesulfonamide |
| Nordihydroguaiaretic acid | 4-[4-(3,4-dihydroxyphenyl)-2,3-dimethylbutyl]benzene-1,2-diol |
| Captan | 2-(trichloromethylsulfanyl)-3a,4,7,7a-tetrahydroisoindole-1,3-dione |
| Benzo[d]thiazole-2(3H)-thione | 3H-1,3-benzothiazole-2-thione |
| Benzothiazyl disulfide | 2-(1,3-benzothiazol-2-yldisulfanyl)-1,3-benzothiazole |
| CBS, N-Cyclohexyl-2-benzothiazolesulfenamide | N-(1,3-benzothiazol-2-ylsulfanyl)cyclohexanamine |
| N-oxydiethylenebenzothiazole-2-sulfenamide | 4-(1,3-benzothiazol-2-ylsulfanyl)morpholine |
| PX-12 | (2-Imidazolyl)(1-methylpropyl)disulfide |
| Carboxypyridine-disulfide | 6-[(5-carboxypyridin-2-yl)disulfanyl]pyridine-3-carboxylic acid |
| IPA-3 | 1,1'-Disulfanediylbis(2-naphthol) |
| Silver-sulfadiazine | silver;(4-aminophenyl)sulfonyl-pyrimidin-2-ylazanide |
| Bonaphthone | 6-Bromonaphthalene-1,2-dione |
| JAK3-inhibitor-V | 1-naphthalen-2-ylprop-2-en-1-one |
| PD119507 | 5-aminoquinolin-8-ol |
| Pyrrolidine-dithiocarbamate | N-pyrrolidin-1-ylcarbamodithioate |
| MIRA-1 | Propionic acid 2,5-dioxo-2,5-dihydro-1H-pyrrol-1-ylmethyl ester |
| Hematoxylin (Hydroxybrazilin) | (6aS,11bR)-7,11b-dihydro-6H-indeno[2,1-c]chromene-3,4,6a,9,10-pentol |
| TAS-103 (dihydrochloride) | 6-[2-(Dimethylamino)ethylamino]-3-hydroxy-7H-indeno[2,1-c]quinolin-7-one dihydrochloride |
| Calpeptin | N2-[(Benzyloxy)carbonyl]-N-[(2S)-1-oxohexan-2-yl]-L-leucinamide |
| Z-DEVD-FM K | N-(Benzyloxycarbonyl)-L-aspartyl-L-glutamyl-L-valyl-L-aspartylfluoromethane |
| Thimerosal | 2-Sulfidobenzoic acid ethylmercury(1+) sodium salt |
| Phenylmercuric acetate | acetyloxy(phenyl)mercury |
| Chloranil | 2,3,5,6-tetrachlorocyclohexa-2,5-diene-1,4-dione |
| SU3327 | 5-(5-Nitrothiazo1-2-ylsulfanyl)-1,3,4-thiadiazol-2-amine |
| Thiram | dimethylcarbamothioylsulfanyl N,N-dimethylcarbamodithioate |
| Disulfiram | Tetraethylthioperoxydicarbonic diamide |
| Pyrithione Zinc | Bis[1-hydroxy-2(1H)-pyiridinethionato]zinc |
| Pyrithione | 1-hydroxypyridine-2-thione |
| Tideglusib | 4-Benzyl-2-(1-naphthyl)-1,2,4-thiadiazolidine-3,5-dione |
| NSC228155 | 4-Nitro-7-[(1-oxidopyridin-2-yl)sulfanyl]-2,1,3-benzoxadiazole |
| Benserazide Hydrochloride | 2-Amino-3-hydroxy-N'-[(2,3,4-trihydroxyphenyl)methyl]propanehydrazide hydrogen chloride |
| ML311 | 7-[(4-Ethylpiperazin-1-yl)[4-(trifluoromethyl)phenyl]methyl]quinolin-8-ol |
| CCG 50014 | 4-(4-Fluorobenzyl)-2-(4-methylphenyl)-1,2,4-thiadiazolidine-3,5-dione |
| SKF-38393 | 2,3,4,5,-Tetrahydro-7,8-dihydroxy-1-phenyl-1H-3-benzazepine |
| Carmofur | 5-Fluoro-N-hexyl-2,4-dioxo-1,2,3,4-tetrahydropyrimidine-1-carboxamide |
| Phenacyl chloride | 2-chloro-1-phenylethanone |
| Felbinac ethyl | [1,1'-Biphenyl]-4-acetic acid ethyl ester |
| Auranofin | (2,3,4,6-Tetra-O-acetyl-1-thio-beta-D-glucopyranosato-S)(triethylphosphine)gold |
| Oltipraz | 4-Methyl-5-(2-pyrazinyl)-3H-1,2-dithiole-3-thione |
| WAY-308264 | 1-(3,5-dimethylphenyl)-3-(1H-1,2,4-triazol-5-ylsulfanyl)pyrrolidine-2,5-dione |
| INCA-6 | Pentacyclo[6.6.6.0(2, 7).0(9,14).0(15,20)] icosa-2(7),4,9,11,13,15,17,19-octaene-3,6-dione |
| Spectrum_000510 | [2,7-dibromo-9-(2-carboxyphenyl)-3-hydroxy-6-oxoxanthen-4-yl]mercury;hydrate |
| HQ-415 | 7-[(3-Ethoxy-4-methoxyphenyl)[(4-methylpyridin-2-yl)amino]methyl]quinolin-8-ol |
| BI-78D3 | 4-(2,3-Dihydro-1,4-benzodioxin-6-yl)-5-(5-nitrothiazol-2-ylsulfanyl)-4H-1,2,4-triazol-3-ol |
| Riodoxol | 2,4,6-Triiodoresorcine |
| PR-619 | 2,6-Diaminopyridine-3,5-diyl bis(thiocyanate) |
| Bronopol | 2-bromo-2-nitropropane-1,3-diol |
| NT157 | 3-(3-Bromo-4,5-dihydroxyphenyl)-N-(3,4,5-trihydroxybenzyl)-2-propenethioamide |
| Apomorphine hydrochloride | (R)-6-Methyl-5,6,6a,7-tetrahydro-4H-dibenzo[de,g]quinoline-10,11-diol hydrochloride hemihydrate |
| PD120911 | 6-[morpholin-4-yl-[4-(trifluoromethyl)phenyl]methyl]-1,3-benzodioxol-5-ol |
| ZM-39923 | 3-[benzyl(propan-2-yl)amino]-1-naphthalen-2-ylpropan-1-one |
| Aldoxorubicin | N'-[1-[4(S)-(3-Amino-2,3,6-trideoxy-alpha-L-lyxo-hexopyranosyloxy)-2(S),5,12-trihydroxy-7-methoxy-6,11-dioxo-1,2,3,4,6,11-hexahydronaphthacen-2-yl]-2-hydroxyethylidene]-6-(2,5-dioxo-2,5-dihydro-1H-pyrrol-1-yl)hexanohydrazide hydrochloride |
| | |
| CR8-(R) | (2R)-2-[[9-propan-2-yl-6-[(4-pyridin-2-ylphenyl)methylamino]purin-2-yl]amino]butan-1-ol |
| Hemin | -(SP-5-13)-Chloro[3,8,13,17-tetramethyl-7,12-divinyl-21H,23H-porphine-2,18-dipropanoato(4-)-kappaN21,kappaN22,kappaN23,kappaN24]dihydrogenoferrate(2-) |
| Tetramethylthiuram monosulfide | dimethylcarbamothioyl N,N-dimethylcarbamodithioate |
| SCH-202676 | N-(2,3-Diphenyl-2,5-dihydro-1,2,4-thiadiazol-5-ylidene)-N-methylamine |
| Dihydrexidine | trans-10,11-Dihydroxy-5,6,6a,7,8,12b-hexahydrobenzo[a]phenanthridine |
| Ro-106-9920 | 6-(Phenylsulfinyl)tetrazolo[1,5-b]pyridazine |
| Vanitiolide | (4-hydroxy-3-methoxyphenyl)-morpholin-4-ylmethanethione |
| Caracemide | N-[(Methylamino)carbonyl]-N-[[(methylamino)carbonyl]oxy]acetamide |
| Sennoside A | (9R)-9-[(9R)-2-carboxy-4-hydroxy-10-oxo-5-[(2S,3R,4S,5S,6R)-3,4,5-trihydroxy-6-(hydroxymethyl)oxan-2-yl]oxy-9H-anthracen-9-yl]-4-hydroxy-10-oxo-5-[(2S,3R,4S,5S,6R)-3,4,5-trihydroxy-6-(hydroxymethyl)oxan-2-yl]oxy-9H-anthracene-2-carboxylic acid |
| Dimercaptosuccinic-acid | 2,3-bis(sulfanyl)butanedioic acid |
| Aurothioglucose | 1-Aurothioglucose |
| SB-268262 | N-methyl-N-(2-methylphenyl)-3-nitro-4-(1,3-thiazol-2-ylsulfinyl)benzamide |
| eseroline-(-) | (3aS,8bR)-3,4,8b-trimethyl-2,3a-dihydro-1H-pyrrolo[2,3-b]indol-7-ol |
| PD096194 | N-(4-bromophenyl)-2-(6-oxo-[1,3]thiazolo[3,2-b][1,2,4]triazol-5-yl)acetamide |
| SB-747651A | 4-[1-ethyl-7-[(piperidin-4-ylamino)methyl]imidazo[4,5-c]pyridin-2-yl]-1,2,5-oxadiazol-3-amine |
| omega-(4-lodophenyl)pentadecanoic acid | 15-(4-iodophenyl)pentadecanoic acid |
| oxyfedrine | 3-[2(R)-Hydroxy-1(S)-methyl-2-phenylethylamino]-1-(3-methoxyphenyl)propan-1-one hydrochloride |
| 4-DAM P | (1,1-dimethylpiperidin-1-ium-4-yl) 2,2-diphenylacetate;iodide |
| Oridonin (Isodonol) | (1S,5S,8R,9S,10S,11R,15S,18R)-9,10,15,18-tetrahydroxy-12,12-dimethyl-6-methylidene-17-oxapentacyclo[7.6.2.15,8.01,11.02,8]octadecan-7-one |
and pharmaceutical salts thereof.

4. A pharmaceutical composition for use in the treatment of a SARS-Cov-2 infection in a subject comprising:
i) A compound selected from:
a compound of formula (I) 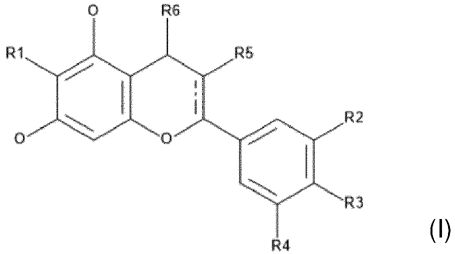 wherein
R1, R2, R3 and R4 are independently selected from OH and H,
R5 is selected from the group consisting of OH, H, alpha-L-rhamnopyranosidyl and 3,4,5-trihydroxybenzoate,
R6 is selected from H and carbonyl,
with the proviso that at least one of the following condition is satisfied:
R1 is OH, or
R2, R3, R4 are OH or
R3 is OH and the fused rings are a chromone;
a compound of formula (II) 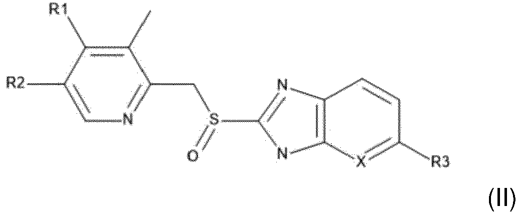 wherein
R1 is selected from the group consisting of OCH₂CF₃, OCH₂CH₂CH₂OCH₃ and OCH₃,
R2 is selected from H and CH₃,
R3 is selected from H and OCH₃,
X is selected from N and C;
a compound of formula (III) 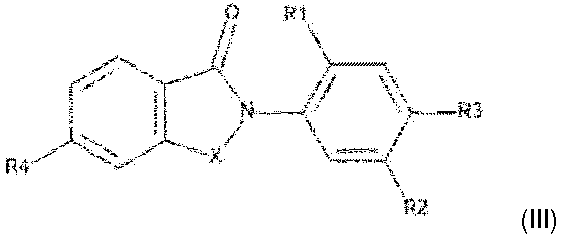 wherein
R1 is selected from H and CH₃,
R2 is selected from the group consisting of H, CH₃ and SO₂N(CH₃)₂,
R3 is selected from H and CH₃,
R4 is selected from H and F,
X is selected from S and Se;
a compound of formula (IV) 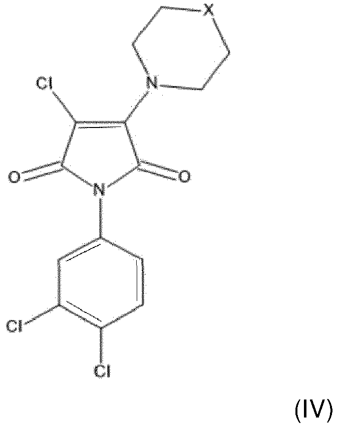 wherein
X is selected from O and NCH₃;
a compound of formula (V) 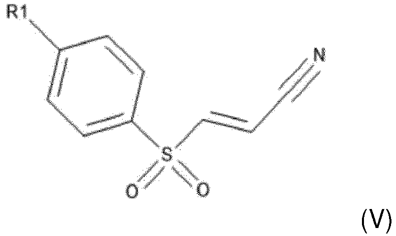 wherein
R1 is selected from CH₃ and C(CH₃)₃;
a compound of formula (VI) 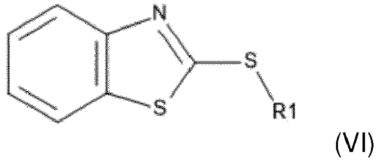 wherein
R1 is selected from the group consisting of H, N-Cyclohexyl, S-benzothiazol-2-yl and morpholin-4-yl;
a compound of formula (VII) 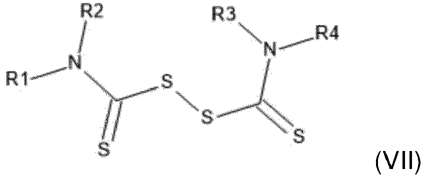 wherein R1, R2, R3 and R4 are independently selected from CH₃ and CH₂CH₃;
a compound of formula (VIII) 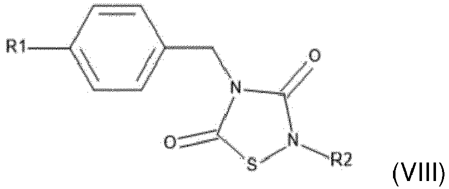 wherein
R1 is selected from H and F,
R2 is selected from 4methylphenyl and 1 naphthalenyl;
a compound of formula (IX) 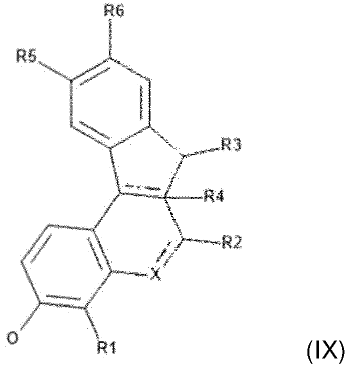 wherein
R1, R4, R5, R6 are independently selected from H and OH,
R2 is selected from H and NCH₂CH₂N(CH₃)₂,
R3 is selected from H and carbonyl,
X is selected from N and O;
the compounds listed in the table below
| **Current Name** | **Chemical Name** |
|---|---|
| TZDZ-8 | 4-Benzyl-2-methyl-1,2,4-thiadiazolidine-3,5-dione |
| CL-17107 | 5-benzyl-3H-1,3,4-thiadiazole-2-thione |
| Tosyl Phenanyl Chloromethyl Ketone | N-[(2S)-5-chloro-3,4-dioxo-1-phenylpentan-2-yl]-4-methylbenzenesulfonamide |
| Nordihydroguaiaretic acid | 4-[4-(3,4-dihydroxyphenyl)-2,3-dimethylbutyl]benzene-1,2-diol |
| Captan | 2-(trichloromethylsulfanyl)-3a,4,7,7a-tetrahydroisoindole-1,3-dione |
| PX-12 | (2-Imidazolyl)(1-methylpropyl)disulfide |
| Carboxypyridine-disulfide | 6-[(5-carboxypyridin-2-yl)disulfanyl]pyridine-3-carboxylic acid |
| IPA-3 | 1,1'-Disulfanediylbis(2-naphthol) |
| Silver-sulfadiazine | silver;(4-aminophenyl)sulfonyl-pyrimidin-2-ylazanide |
| Bonaphthone | 6-Bromonaphthalene-1,2-dione |
| JAK3-inhibitor-V | 1-naphthalen-2-ylprop-2-en-1-one |
| PD119507 | 5-aminoquinolin-8-ol |
| Pyrrolidine-dithiocarbamate | N-pyrrolidin-1-ylcarbamodithioate |
| MIRA-1 | Propionic acid 2,5-dioxo-2,5-dihydro-1H-pyrrol-1-ylmethyl ester |
| Calpeptin | N2-[(Benzyloxy)carbonyl]-N-[(2S)-1-oxohexan-2-yl]-L-leucinamide |
| Z-DEVD-FM K | N-(Benzyloxycarbonyl)-L-aspartyl-L-glutamyl-L-valyl-L-aspartylfluoromethane |
| Thimerosal | 2-Sulfidobenzoic acid ethylmercury(1+) sodium salt |
| Phenylmercuric acetate | acetyloxy(phenyl)mercury |
| Chloranil | 2,3,5,6-tetrachlorocyclohexa-2,5-diene-1,4-dione |
| SU3327 | 5-(5-Nitrothiazol-2-ylsulfanyl)-1,3,4-thiadiazol-2-amine |
| Pyrithione Zinc | Bis[1-hydroxy-2(1H)-pyiridinethionato]zinc |
| Pyrithione | 1-hydroxypyridine-2-thione |
| NSC228155 | 4-Nitro-7-[(1-oxidopyridin-2-yl)sulfanyl]-2,1,3-benzoxadiazole |
| Benserazide Hydrochloride | 2-Amino-3-hydroxy-N'-[(2,3,4-trihydroxyphenyl)methyl]propanehydrazide hydrogen chloride |
| ML311 | 7-[(4-Ethylpiperazin-1-yl)[4-(trifluoromethyl)phenyl]methyl]quinolin-8-ol |
| SKF-38393 | 2,3,4,5,-Tetrahydro-7,8-dihydroxy-1-phenyl-1H-3-benzazepine |
| Carmofur | 5-Fluoro-N-hexyl-2,4-dioxo-1,2,3,4-tetrahydropyrimidine-1-carboxamide |
| Phenacyl chloride | 2-chloro-1-phenylethanone |
| Felbinac ethyl | [1,1'-Biphenyl]-4-acetic acid ethyl ester |
| Auranofin | (2,3,4,6-Tetra-O-acetyl-1-thio-beta-D-glucopyranosato-S)(triethylphosphine)gold |
| Oltipraz | 4-Methyl-5-(2-pyrazinyl)-3H-1,2-dithiole-3-thione |
| WAY-308264 | 1-(3,5-dimethylphenyl)-3-(1H-1,2,4-triazol-5-ylsulfanyl)pyrrolidine-2,5-dione |
| INCA-6 | Pentacyclo[6.6.6.0(2, 7).0(9,14).0(15,20)] icosa-2(7),4,9,11,13,15,17,19-octaene-3,6-dione |
| Spectrum_000510 | [2,7-dibromo-9-(2-carboxyphenyl)-3-hydroxy-6-oxoxanthen-4-yl]mercury;hydrate |
| HQ-415 | 7-[(3-Ethoxy-4-methoxyphenyl)[(4-methylpyridin-2-yl)amino]methyl]quinolin-8-ol |
| BI-78D3 | 4-(2,3-Dihydro-1,4-benzodioxin-6-yl)-5-(5-nitrothiazol-2-ylsulfanyl)-4H-1,2,4-triazol-3-ol |
| Riodoxol | 2,4,6-Triiodoresorcine |
| PR-619 | 2,6-Diaminopyridine-3,5-diyl bis(thiocyanate) |
| Bronopol | 2-bromo-2-nitropropane-1,3-diol |
| NT157 | 3-(3-Bromo-4,5-dihydroxyphenyl)-N-(3,4,5-trihydroxybenzyl)-2-propenethioamide |
| Apomorphine hydrochloride | (R)-6-Methyl-5,6,6a,7-tetrahydro-4H-dibenzo[de,g]quinoline-10,11-diolhydrochloride hemihydrate |
| PD120911 | 6-[morpholin-4-yl-[4-(trifluoromethyl)phenyl]methyl]-1,3-benzodioxol-5-ol |
| ZM-39923 | 3-[benzyl(propan-2-yl)amino]-1-naphthalen-2-ylpropan-1-one |
| Aldoxorubicin | N'-[1-[4(S)-(3-Amino-2,3,6-trideoxy-alpha-L-lyxo-hexopyranosyloxy)-2(S),5,12-trihydroxy-7-methoxy-6,11-dioxo-1,2,3,4,6,11-hexahydronaphthacen-2-yl]-2-hydroxyethylidene]-6-(2,5-dioxo-2,5-dihydro-1H-pyrrol-1-yl)hexanohydrazide hydrochloride |
| CR8-(R) | (2R)-2-[[9-propan-2-yl-6-[(4-pyridin-2-ylphenyl)methylamino]purin-2-yl]amino]butan-1-ol |
| Hemin | - (SP-5-13)-Chloro[3,8,13,17-tetramethyl-7,12-divinyl-21H,23H-porphine-2,18-dipropanoato(4-)-kappaN21,kappaN22,kappaN23,kappaN24]dihydrogenoferrate(2-) |
| Tetramethylthiuram monosulfide | dimethylcarbamothioyl N,N-dimethylcarbamodithioate |
| SCH-202676 | N-(2,3-Diphenyl-2,5-dihydro-1,2,4-thiadiazol-5-ylidene)-N-methylamine |
| Dihydrexidine | trans-10,11-Dihydroxy-5,6,6a,7,8,12b-hexahydrobenzo[a]phenanthridine |
| Ro-106-9920 | 6-(Phenylsulfinyl)tetrazolo[1,5-b]pyridazine |
| Vanitiolide | (4-hydroxy-3-methoxyphenyl)-morpholin-4-ylmethanethione |
| Caracemide | N-[(Methylamino)carbonyl]-N-[[(methylamino)carbonyl]oxy]acetamide |
| Sennoside A | (9R)-9-[(9R)-2-carboxy-4-hydroxy-10-oxo-5-[(2S,3R,4S,5S,6R)-3,4,5-trihydroxy-6-(hydroxymethyl)oxan-2-yl]oxy-9H-anthracen-9-yl]-4-hydroxy-10-oxo-5-[(2S,3R,4S,5S,6R)-3,4,5-trihydroxy-6-(hydroxymethyl)oxan-2-yl]oxy-9H-anthracene-2-carboxylic acid |
| Dimercaptosuccinic-acid | 2,3-bis(sulfanyl)butanedioic acid |
| Aurothioglucose | 1-Aurothioglucose |
| SB-268262 | N-methyl-N-(2-methylphenyl)-3-nitro-4-(1,3-thiazol-2-ylsulfinyl)benzamide |
| eseroline-(-) | (3aS,8bR)-3,4,8b-trimethyl-2,3a-dihydro-1H-pyrrolo[2,3-b]indol-7-ol |
| PD096194 | N-(4-bromophenyl)-2-(6-oxo-[1,3]thiazolo[3,2-b][1,2,4]triazol-5-yl)acetamide |
| SB-747651A | 4-[1-ethyl-7-[(piperidin-4-ylamino)methyl]imidazo[4,5-c]pyridin-2-yl]-1,2,5-oxadiazol-3-amine |
| omega-(4-lodophenyl)pentadecanoic acid | 15-(4-iodophenyl)pentadecanoic acid |
| oxyfedrine | 3-[2(R)-Hydroxy-1(S)-methyl-2-phenylethylamino]-1-(3-methoxyphenyl)propan-1-one hydrochloride |
| 4-DAM P | (1,1-dimethylpiperidin-1-ium-4-yl) 2,2-diphenylacetate;iodide |
| Oridonin (Isodonol) | (1S,5S,8R,9S,10S,11R,15S,18R)-9,10,15,18-tetrahydroxy-12,12-dimethyl-6-methylidene-17-oxapentacyclo[7.6.2.15,8.01,11.02,8]octadecan-7-one |
and pharmaceutically acceptable salts thereof, and
ii) at least one inert pharmaceutically acceptable excipient.

5. A pharmaceutical composition for use according to claim 4, wherein
said compound of formula (I) is selected from the group consisting of 5,6,7-Trihydroxy-2-phenyl-4H-1-benzopyran-4-one, (+)-3(R),5,7-Trihydroxy-2(R)-(3,4,5-trihydroxyphenyl)-3,4-dihydro-2H-1-benzopyran-4-one, 3,5,7-Trihydroxy-2-(3,4,5-trihydroxyphenyl)-4H-1-benzopyran-4-one, 4',5,7-Trihydroxyflavone and 5,7-Dihydroxy-4-oxo-2-(3,4,5-trihydroxyphenyl)-4H-1-benzopyran-3-yl alpha-L-rhamnopyranoside, (-)-3,4,5-Trihydroxybenzoic acid 5,7-dihydroxy-2(R)-(3,4,5-trihydroxyphenyl)-3,4-dihydro-2H-1-benzopyran-3(R)-yl ester
or
said compound of formula (II) is selected from the group consisting of 2-([[3-Methyl-4-(2,2,2-trifluoroethoxy)pyridin-2-yl]methyl]sulfinyl)-1H-benzimidazole, 2-[4-(3-Methoxypropoxy)-3-methylpyridin-2-ylmethylsulfinyl]benzimidazol-1-ide sodium salt and (±)-5-Methoxy-2-(4-methoxy-3,5-dimethylpyridin-2-ylmethylsulfinyl)imidazo[4,5-b]pyridine,
or
said compound of formula (III) is selected from the group consisting of 2-Phenyl-1,2-benzisoselenazol-3(2H)-one, 2-(2,5-Dimethylphenyl)-6-fluoro-1,2-benzothia zol-3(2H)-one, 2-(4-methylphenyl)-1,2-benzothiazol-3-one, N,N-dimethyl-3-(3-oxo-1,2-benzothiazol-2-yl)benzenesulfonamide and 2-phenyl-1,2-benzothiazol-3-one,
or
said compound of formula (IV) is selected from 3-chloro-1-(3,4-dichlorophenyl)-4-morpholin-4-ylpyrrole-2,5-dione and 3-chloro-1-(3,4-dichlorophenyl)-4-(4-methylpiperazin-1-yl)pyrrole-2,5-dione,
or
said compound of formula (V) is selected from 3-(4-Methylphenylsulfonyl)-2-propenenitrile and 3-(4-tert-Butylphenylsulfonyl)-2(E)-propenenitrile,
or
said compound of formula (VI) is selected from the group consisting of 3H-1,3-benzothiazole-2-thione, N-(1,3-benzothiazol-2-ylsulfanyl)cyclohexanamine, 4-(1,3-benzothiazol-2-ylsulfanyl)morpholine and 2-(1,3-benzothiazol-2-yldisulfanyl)-1,3-benzothiazole,
or
said compound of formula (VII) is selected from dimethylcarbamothioylsulfanyl N,N-dimethylcarbamodithioate and Tetraethylthioperoxydicarbonic diamide,
or
said compound of formula (VIII) is selected from 4-Benzyl-2-(1-naphthyl)-1,2,4-thiadiazolidine-3,5-dione and 4-(4-Fluorobenzyl)-2-(4-methylphenyl)-1,2,4-thiadiazolidine-3,5-dione,
or
said compound of formula (IX) is selected from (6aS,11bR)-7,11b-dihydro-6H-indeno[2,1-c]chromene-3,4,6a,9,10-pentol and 6-[2-(Dimethylamino)ethylamino]-3-hydroxy-7H-indeno[2,1-c]quinolin-7-one dihydrochloride.

6. A pharmaceutical composition for use according to claim 4 or claim 5, wherein said compound is selected from the compounds listed in the table below:
| **Current Name** | **Chemical Name** |
|---|---|
| Baicalein | 5,6,7-Trihydroxy-2-phenyl-4H-1-benzopyran-4-one |
| Dihydromyricetin | (+)-3(R),5,7-Trihydroxy-2(R)-(3,4,5-trihydroxyphenyl)-3,4-dihydro-2H-1-benzopyran-4-one |
| Myricetin | 3,5,7-Trihydroxy-2-(3,4,5-trihydroxyphenyl)-4H-1-benzopyran-4-one |
| Apigenin | 4',5,7-Trihydroxyflavone |
| Myricitrin | 5,7-Dihydroxy-4-oxo-2-(3,4,5-trihydroxyphenyl)-4H-1-benzopyran-3-yl alpha-L-rhamnopyranoside |
| (-)-Epigallocatechin Gallate | (-)-3,4,5-Trihydroxybenzoic acid 5,7-dihydroxy-2(R)-(3,4,5-trihydroxyphenyl)-3,4-dihydro-2H-1-benzopyran-3(R)-yl ester |
| Lansoprazole | 2-([[3-Methyl-4-(2,2,2-trifluoroethoxy)pyridin-2-yl]methyl]sulfinyl)-1H-benzimidazole |
| Rabeprazole (sodium) | 2-[4-(3-Methoxypropoxy)-3-methylpyridin-2-ylmethylsulfinyl]benzimidazol-1-ide sodium salt |
| Tenatoprazole | (±)-5-Methoxy-2-(4-methoxy-3,5-dimethylpyridin-2-ylmethylsulfinyl)imidazo[4,5-b]pyridine |
| Ebselen | 2-Phenyl-1,2-benzisoselenazol-3(2H)-one |
| MLS-0315771 | 2-(2,5-Dimethylphenyl)-6-fluoro-1,2-benzothia zol-3(2H)-one |
| PBIT | 2-(4-methylphenyl)-1,2-benzothiazol-3-one |
| ML345 Analog | N,N-dimethyl-3-(3-oxo-1,2-benzothiazol-2-yl)benzenesulfonamide |
| PD086290 | 2-phenyl-1,2-benzothiazol-3-one |
| RI-1 | 3-chloro-1-(3,4-dichlorophenyl)-4-morpholin-4-ylpyrrole-2,5-dione |
| PD119792 | 3-chloro-1-(3,4-dichlorophenyl)-4-(4-methylpiperazin-1-yl)pyrrole-2,5-dione |
| Bay-11-7082 | 3-(4-Methylphenylsulfonyl)-2-propenenitrile |
| Bay-11-7085 | 3-(4-tert-Butylphenylsulfonyl)-2(E)-propenenitrile |
| TZDZ-8 | 4-Benzyl-2-methyl-1,2,4-thiadiazolidine-3,5-dione |
| CL-17107 | 5-benzyl-3H-1,3,4-thiadiazole-2-thione |
| Tosyl Phenanyl Chloromethyl Ketone | N-[(2S)-5-chloro-3,4-dioxo-1-phenylpentan-2-yl]-4-methylbenzenesulfonamide |
| Nordihydroguaiaretic acid | 4-[4-(3,4-dihydroxyphenyl)-2,3-dimethylbutyl]benzene-1,2-diol |
| Captan | 2-(trichloromethylsulfanyl)-3a,4,7,7a-tetrahydroisoindole-1,3-dione |
| Benzo[d]thiazole-2(3H)-thione | 3H-1,3-benzothiazole-2-thione |
| Benzothiazyl disulfide | 2-(1,3-benzothiazol-2-yldisulfanyl)-1,3-benzothiazole |
| CBS, N-Cyclohexyl-2-benzothiazolesulfenamide | N-(1,3-benzothiazol-2-ylsulfanyl)cyclohexanamine |
| N-oxydiethylenebenzothiazole-2-sulfenamide | 4-(1,3-benzothiazol-2-ylsulfanyl)morpholine |
| PX-12 | (2-Imidazolyl)(1-methylpropyl)disulfide |
| Carboxypyridine-disulfide | 6-[(5-carboxypyridin-2-yl)disulfanyl]pyridine-3-carboxylic acid |
| IPA-3 | 1,1'-Disulfanediylbis(2-naphthol) |
| Silver-sulfadiazine | silver;(4-aminophenyl)sulfonyl-pyrimidin-2-ylazanide |
| Bonaphthone | 6-Bromonaphthalene-1,2-dione |
| JAK3-inhibitor-V | 1-naphthalen-2-ylprop-2-en-1-one |
| PD119507 | 5-aminoquinolin-8-ol |
| Pyrrolidine-dithiocarbamate | N-pyrrolidin-1-ylcarbamodithioate |
| MIRA-1 | Propionic acid 2,5-dioxo-2,5-dihydro-1H-pyrrol-1-ylmethyl ester |
| Hematoxyl in (Hydroxybrazilin) | (6aS,11bR)-7,11b-dihydro-6H-indeno[2,1-c]chromene-3,4,6a,9,10-pentol |
| TAS-103 (dihydrochloride) | 6-[2-(Dimethylamino)ethylamino]-3-hydroxy-7H-indeno[2,1-c]quinolin-7-one dihydrochloride |
| Cal pepti n | N2-[(Benzyloxy)carbonyl]-N-[(2S)-1-oxohexan-2-yl]-L-leucinamide |
| Z-DEVD-FM K | N-(Benzyloxycarbonyl)-L-aspartyl-L-glutamyl-L-valyl-L-aspartylfluoromethane |
| Thimerosal | 2-Sulfidobenzoic acid ethylmercury(1+) sodium salt |
| Phenylmercuric acetate | acetyloxy(phenyl)mercury |
| Chloranil | 2,3,5,6-tetrachlorocyclohexa-2,5-diene-1,4-dione |
| SU3327 | 5-(5-Nitrothiazol-2-ylsulfanyl)-1,3,4-thiadiazol-2-amine |
| Thiram | dimethylcarbamothioylsulfanyl N,N-dimethylcarbamodithioate |
| Disulfiram | Tetraethylthioperoxydicarbonic diamide |
| Pyrithione Zinc | Bis[1-hydroxy-2(1H)-pyiridinethionato]zinc |
| Pyrithione | 1-hydroxypyridine-2-thione |
| Tideglusib | 4-Benzyl-2-(1-naphthyl)-1,2,4-thiadiazolidine-3,5-dione |
| NSC228155 | 4-Nitro-7-[(1-oxidopyridin-2-yl)sulfanyl]-2,1,3-benzoxadiazole |
| Benserazide Hydrochloride | 2-Amino-3-hydroxy-N'-[(2,3,4-trihydroxyphenyl)methyl]propanehydrazide hydrogen chloride |
| ML311 | 7-[(4-Ethylpiperazin-1-yl)[4-(trifluoromethyl)phenyl]methyl]quinolin-8-ol |
| CCG 50014 | 4-(4-Fluorobenzyl)-2-(4-methylphenyl)-1,2,4-thiadiazolidine-3,5-dione |
| SKF-38393 | 2,3,4,5,-Tetrahydro-7,8-dihydroxy-1-phenyl-1H-3-benzazepine |
| Carmofur | 5-Fluoro-N-hexyl-2,4-dioxo-1,2,3,4-tetrahydropyrimidine-1-carboxamide |
| Phenacyl chloride | 2-chloro-1-phenylethanone |
| Felbinac ethyl | [1,1'-Biphenyl]-4-acetic acid ethyl ester |
| Auranofin | (2,3,4,6-Tetra-O-acetyl-1-thio-beta-D-glucopyranosato-S)(triethylphosphine)gold |
| Oltipraz | 4-Methyl-5-(2-pyrazinyl)-3H-1,2-dithiole-3-thione |
| WAY-308264 | 1-(3,5-dimethylphenyl)-3-(1H-1,2,4-triazol-5-ylsulfanyl)pyrrolidine-2,5-dione |
| INCA-6 | Pentacyclo[6.6.6.0(2,7).0(9,14).0(15,20)]icosa-2(7),4,9,11,13,15,17,19-octaene-3,6-dione |
| Spectrum_000510 | [2,7-dibromo-9-(2-carboxyphenyl)-3-hydroxy-6-oxoxanthen-4-yl]mercury;hydrate |
| HQ-415 | 7-[(3-Ethoxy-4-methoxyphenyl)[(4-methylpyridin-2-yl)amino]methyl]quinolin-8-ol |
| BI-78D3 | 4-(2,3-Dihydro-1,4-benzodioxin-6-yl)-5-(5-nitrothiazol-2-ylsulfanyl)-4H-1,2,4-triazol-3-ol |
| Riodoxol | 2,4,6-Triiodoresorcine |
| PR-619 | 2,6-Diaminopyridine-3,5-diyl bis(thiocyanate) |
| Bronopol | 2-bromo-2-nitropropane-1,3-diol |
| NT157 | 3-(3-Bromo-4,5-dihydroxyphenyl)-N-(3,4,5-trihydroxybenzyl)-2-propenethioamide |
| Apomorphine hydrochloride | (R)-6-Methyl-5,6,6a,7-tetrahydro-4H-dibenzo[de,g]quinoline-10,11-diol hydrochloride hemihydrate |
| PD120911 | 6-[morpholin-4-yl-[4-(trifluoromethyl)phenyl]methyl]-1,3-benzodioxol-5-ol |
| ZM-39923 | 3-[benzyl(propan-2-yl)amino]-1-naphthalen-2-ylpropan-1-one |
| Aldoxorubicin | N'-[1-[4(S)-(3-Amino-2,3,6-trideoxy-alpha-L-lyxo-hexopyranosyloxy)-2(S),5,12-trihydroxy-7-methoxy-6,11-dioxo-1,2,3,4,6,11-hexahydronaphthacen-2-yl]-2-hydroxyethylidene]-6-(2,5-dioxo-2,5-dihydro-1H-pyrrol-1-yl)hexanohydrazide hydrochloride |
| | |
| CR8-(R) | (2R)-2-[[9-propan-2-yl-6-[(4-pyridin-2-ylphenyl)methylamino]purin-2-yl]amino]butan-1-ol |
| Hemin | -(SP-5-13)-Chloro[3,8,13,17-tetramethyl-7,12-divinyl-21H,23H-porphine-2,18-dipropanoato(4-)-kappaN21,kappaN22,kappaN23,kappaN24]dihydrogenoferrate(2-) |
| Tetramethylthiuram monosulfide | dimethylcarbamothioyl N,N-dimethylcarbamodithioate |
| SCH-202676 | N-(2,3-Diphenyl-2,5-dihydro-1,2,4-thiadiazol-5-ylidene)-N-methylamine |
| Dihydrexidine | trans-10,11-Dihydroxy-5,6,6a,7,8,12b-hexahydrobenzo[a]phenanthridine |
| Ro-106-9920 | 6-(Phenylsulfinyl)tetrazolo[1,5-b]pyridazine |
| Vanitiolide | (4-hydroxy-3-methoxyphenyl)-morpholin-4-ylmethanethione |
| Caracemide | N-[(Methylamino)carbonyl]-N-[[(methylamino)carbonyl]oxy]acetamide |
| Sennoside A | (9R)-9-[(9R)-2-carboxy-4-hydroxy-10-oxo-5-[(2S,3R,4S,5S,6R)-3,4,5-trihydroxy-6-(hydroxymethyl)oxan-2-yl]oxy-9H-anthracen-9-yl]-4-hydroxy-10-oxo-5-[(2S,3R,4S,5S,6R)-3,4,5-trihydroxy-6-(hydroxymethyl)oxan-2-yl]oxy-9H-anthracene-2-carboxylic acid |
| Dimercaptosuccinic-acid | 2,3-bis(sulfanyl)butanedioic acid |
| Aurothioglucose | 1-Aurothioglucose |
| SB-268262 | N-methyl-N-(2-methylphenyl)-3-nitro-4-(1,3-thiazol-2-ylsulfinyl)benzamide |
| eseroline-(-) | (3aS,8bR)-3,4,8b-trimethyl-2,3a-dihydro-1H-pyrrolo[2,3-b]indol-7-ol |
| PD096194 | N-(4-bromophenyl)-2-(6-oxo-[1,3]thiazolo[3,2-b][1,2,4]triazol-5-yl)acetamide |
| SB-747651A | 4-[1-ethyl-7-[(piperidin-4-ylamino)methyl]imidazo[4,5-c]pyridin-2-yl]-1,2,5-oxadiazol-3-amine |
| omega-(4-lodophenyl)pentadecanoic acid | 15-(4-iodophenyl)pentadecanoic acid |
| oxyfedrine | 3-[2(R)-Hydroxy-1(S)-methyl-2-phenylethylamino]-1-(3-methoxyphenyl)propan-1-one hydrochloride |
| 4-DAM P | (1,1-dimethylpiperidin-1-ium-4-yl) 2, 2-di phenylacetate; iodide |
| Oridonin (Isodonol) | (1S,5S,8R,9S,10S,11R,15S,18R)-9,10,15,18-tetrahydroxy-12,12-dimethyl-6-methylidene-17-oxapentacyclo[7.6.2.15,8.01,11.02,8]octadecan-7-one |

7. A compound for use according to any one of claims 1 to 3 or a pharmaceutical composition for use according to any one of claims 4 to 6, wherein said compound or composition is administered orally.

8. A pharmaceutical composition for use according to any one of claims 4 to 7, in form of a table or capsule.

9. A compound for use according to any one of claims 1 to 3 or a pharmaceutical composition for use according to any one of claims 4 to 6, wherein said compound or composition is administered parenterally.

10. A compound or pharmaceutical composition for use according to claim 9, wherein said compound or composition is administered by intraveneous administration or continuous infusion.
